(19) **Europäisches Patentamt / European Patent Office / Office européen des brevets**

(11) **EP 4 344 623 A1**

(12) **EUROPEAN PATENT APPLICATION**
published in accordance with Art. 153(4) EPC

(43) Date of publication:
**03.04.2024 Bulletin 2024/14**

(21) Application number: **22836788.4**

(22) Date of filing: **29.06.2022**

(51) International Patent Classification (IPC):
**A61B 5/0537** (2021.01)   **A61B 5/053** (2021.01)

(52) Cooperative Patent Classification (CPC):
**A61B 5/00; A61B 5/053; A61B 5/0537**

(86) International application number:
**PCT/CN2022/102467**

(87) International publication number:
**WO 2023/280035 (12.01.2023 Gazette 2023/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.07.2021 CN 202110770421**

(71) Applicant: **Huawei Technologies Co., Ltd. Shenzhen, Guangdong 518129 (CN)**

(72) Inventors:
- **ZHAO, Shuai**
  **Shenzhen, Guangdong 518129 (CN)**
- **YAN, Jiabing**
  **Shenzhen, Guangdong 518129 (CN)**
- **YANG, Bin**
  **Shenzhen, Guangdong 518129 (CN)**
- **REN, Huichao**
  **Shenzhen, Guangdong 518129 (CN)**

(74) Representative: **Pfenning, Meinig & Partner mbB Patent- und Rechtsanwälte Theresienhöhe 11a 80339 München (DE)**

(54) **PHYSIOLOGICAL PARAMETER MEASUREMENT METHOD AND WEARABLE DEVICE**

(57) A wearable device is provided. Two groups of electrodes are disposed on a device body of the wearable device. One group of electrodes are disposed on a rear part of the device body, and the other group of electrodes are disposed on a lateral side of the device body. The two groups of electrodes on the wearable device may measure physiological parameters, such as a liver risk level of a user, to implement accurate and convenient measurement of the physiological parameters, such as the liver risk level of the user, and improve user experience.

FIG. 12

## Description

This application claims priority to Chinese Patent Application No. 202110770421.2, filed with the China National Intellectual Property Administration on July 7, 2021 and entitled "PHYSIOLOGICAL PARAMETER MEASUREMENT METHOD AND WEARABLE DEVICE", which is incorporated herein by reference in its entirety.

## TECHNICAL FIELD

This application relates to the field of terminal technologies, and in particular, to a physiological parameter measurement method and a wearable device.

## BACKGROUND

With continuous improvement of living standards, fatty liver (fatty liver) disease has become the leading hepatic disease of humans, and the morbidity rate gradually increases. The fatty liver disease is a result of liver lesions caused by a build-up of excessive fat in hepatocytes. Generally, when the hepatic tissue has more than 5% steatosis, it can be diagnosed as the fatty liver disease. Therefore, liver fat is an important metric in fatty liver disease detection. However, current liver examination processes are excessively complex, and examination devices are not portable. This is very unfriendly to users. Therefore, how to provide a device that can accurately and conveniently measure a liver risk level of a user is a technical problem urgently to be resolved currently.

## SUMMARY

This application provides a physiological parameter measurement method, a wearable device, a computer storage medium, and a computer program product that includes instructions, to accurately and conveniently examine the liver of a user.

According to a first aspect, this application provides a physiological parameter measurement method. The method is applied to a wearable device, and the wearable device includes a device body, where a display screen, a first group of electrodes, and a second group of electrodes are disposed on the device body, the first group of electrodes are located on a side that is on the device body but is opposite to the display screen, and the second group of electrodes are located on a lateral side of the device body. The method includes: detecting a first operation; displaying a first user interface in response to the first operation, where the first user interface indicates a user to respectively contact a first part and a second part with the first group of electrodes and the second group of electrodes, to conduct the first group of electrodes and the second group of electrodes; generating a first current when the first group of electrodes and the second group of electrodes are conducted, where the first current has a first current value; determining a first voltage value between the first group of electrodes and the second group of electrodes; determining a first physiological parameter based on the first current value and the first voltage value; displaying a second user interface, where the second user interface indicates the user to respectively contact the first part and a third part with the first group of electrodes and the second group of electrodes, to conduct the first group of electrodes and the second group of electrodes; generating a second current when the first group of electrodes and the second group of electrodes are conducted, where the second current has a second current value; determining a second voltage value between the first group of electrodes and the second group of electrodes; determining a second physiological parameter based on the second current value and the second voltage value; and determining a third physiological parameter based on the first physiological parameter and the second physiological parameter. In this way, the wearable device can accurately and conveniently measure physiological parameters such as liver impedance of the user, to determine a liver risk level of the user based on the measured liver impedance.

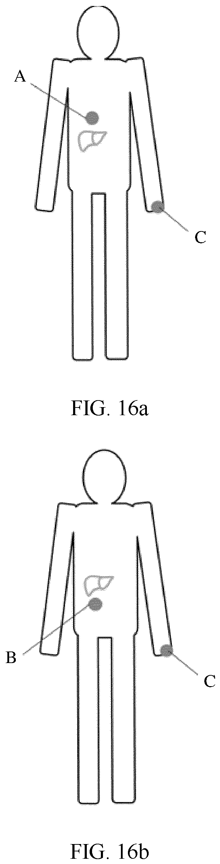

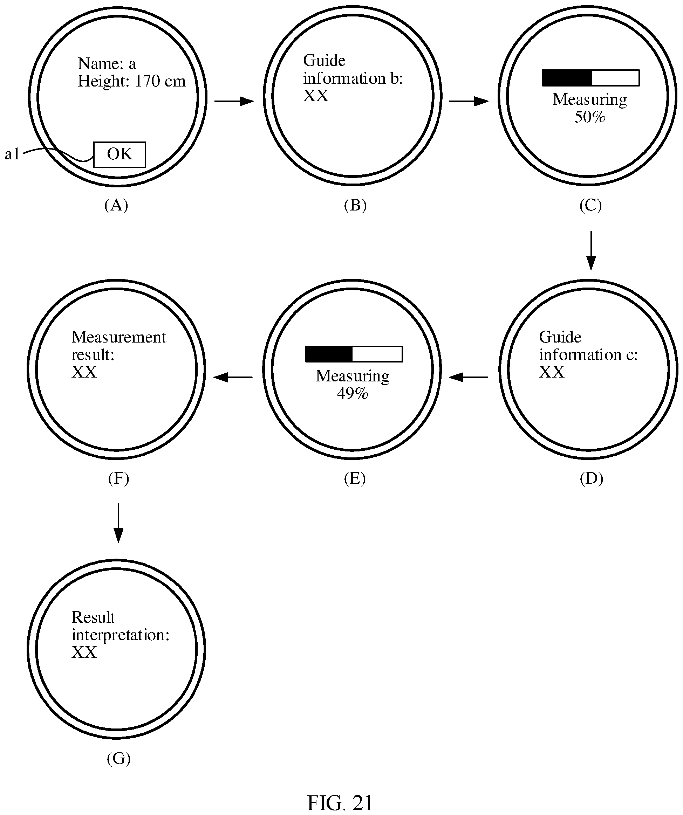

For example, the first part may be an area C shown in FIG. 16a, the second part may be an area A shown in FIG. 16a, and the third part may be an area B shown in FIG. 16b. For example, the first physiological parameter may be impedance from an upper part of the liver to the left upper limb, the second physiological parameter may be impedance from a lower part of the liver to the left upper limb, and the third physiological parameter may be liver impedance. The first operation may be an operation that the user selects to examine the liver, for example, an operation that the user selects an area a1 in (A) in FIG. 21.

In a possible implementation, after determining the second physiological parameter based on the second current value and the second voltage value, the method further includes: displaying a third user interface, where the third user interface indicates the user to respectively contact the second part and the third part with the first group of electrodes and the second group of electrodes, to conduct the first group of electrodes and the second group of electrodes; generating a third current when the first group of electrodes and the second group of electrodes are conducted, where the third current has a third current value; determining a third voltage value between the first group of electrodes and the second group of electrodes; determining a fourth physiological parameter based on the third current value and the third voltage value; determining the third physiological parameter based on the first physiological parameter and the second physiological parameter. The method specifically includes: determining the third physiological parameter based on the first physiological parameter, the second physiological parameter, and the fourth physiological parameter.

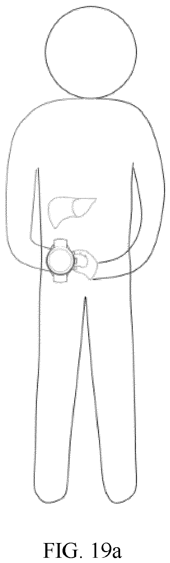

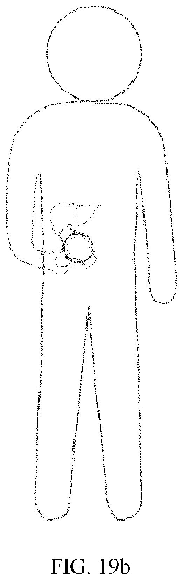

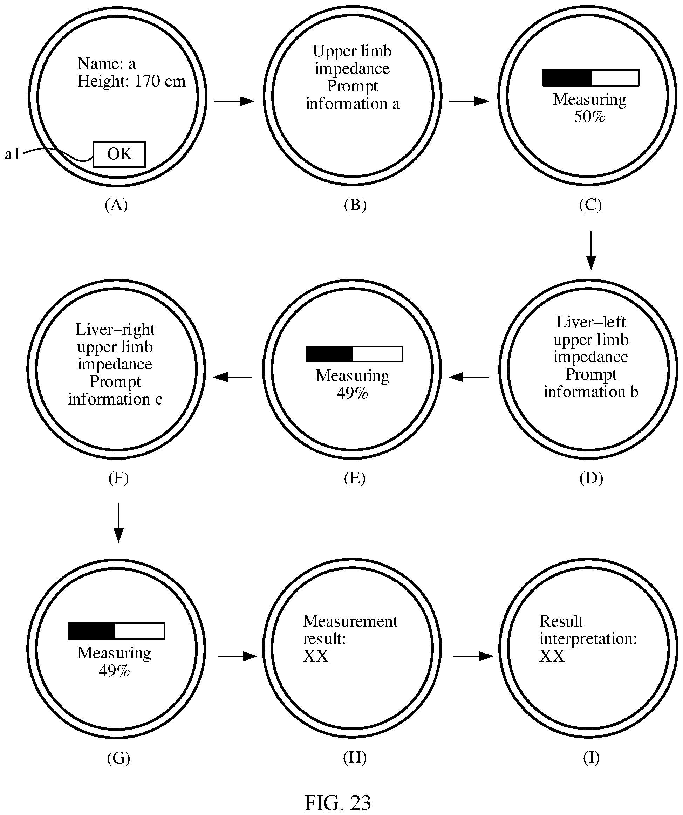

For example, the first part may be a finger of the left hand of the user shown in FIG. 19a, the second part may be a finger of the right hand of the user shown in FIG. 19a, and the third part may be a position below the liver shown in FIG. 19b. For example, the first physiological parameter may be upper limb impedance, the second physiological parameter may be impedance from the liver to the left upper limb, the third physiological parameter may be impedance from the liver to the right upper limb, and the fourth physiological parameter may be liver impedance. The first operation may be an operation that the user selects to examine the liver, for example, an operation that the user selects an area a1 in (A) in FIG. 23.

In a possible implementation, the method includes: generating a target current, where the target current has a target current value; determining a target voltage value between the first group of electrodes and the second group of electrodes; and determining a target physiological parameter based on the target current value and the target voltage value. The method further includes: generating currents of m frequencies, where m is a positive integer greater than or equal to 2, currents of different frequencies are generated at different moments, and current values of the currents of different frequencies each are the target current value; determining voltage values between the first group of electrodes and the second group of electrodes under the currents of different frequencies, to obtain m voltage values, where one voltage value corresponds to a current of one frequency; and determining the target physiological parameter based on the current value of the target current and the m voltage values. The target current is the first current, the target current value is the first current value, the target voltage value is the first voltage value, and the target physiological parameter is the first physiological parameter; or the target current is the second current, the target current value is the second current value, the target voltage value is the second voltage value, and the target physiological parameter is the second physiological parameter; or the target current is the third current, the target current value is the third current value, the target voltage value is the third voltage value, and the target physiological parameter is the fourth physiological parameter. In this way, a physiological parameter can be measured from a plurality of dimensions by using a plurality of currents of different frequencies, thereby improving accuracy of measuring the third physiological parameter.

In a possible implementation, the second group of electrodes are integrated in a physical button on the lateral side of the device body.

In a possible implementation, the first group of electrodes include one electrode, and the second group of electrodes include one electrode; or the first group of electrodes include two electrodes, and the second group of electrodes include two electrodes.

In a possible implementation, the second group of electrodes include a first electrode and a second electrode, and the first electrode and the second electrode are located on a same side of the device body.

According to a second aspect, this application provides a physiological parameter measurement method, where the method is applied to a wearable device. The wearable device includes a device body, the device body includes a display screen, a first group of electrodes, and a second group of electrodes, the first group of electrodes are located on a side that is on the device body but is opposite to the display screen, and the second group of electrodes are located on a lateral side of the device body. The method includes: detecting a first operation; displaying a first user interface in response to the first operation, where the first user interface indicates a user to respectively contact a first part and a second part with a first group of electrodes and a second group of electrodes, to conduct the first group of electrodes and the second group of electrodes; generating a first current when the first group of electrodes and the second group of electrodes are conducted, where the first current has a first current value; determining a first voltage value between the first group of electrodes and the second group of electrodes; determining a first physiological parameter based on the first current value and the first voltage value; determining n second physiological parameters based on the first physiological parameter, where n is a positive integer greater than or equal to 1; and obtaining a third physiological parameter based on the n second physiological parameters. In this way, the wearable device can accurately and conveniently measure physiological parameters such as liver impedance of the user, to determine a liver risk level of the user based on the measured liver impedance.

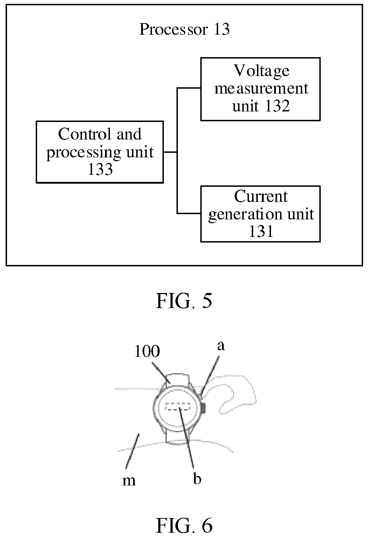

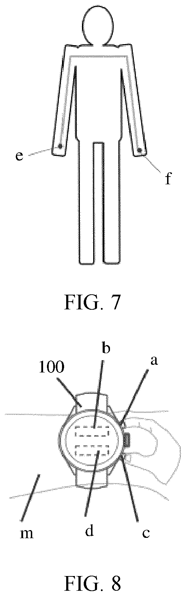

For example, the first part may be an arm of the user shown in FIG. 6 or FIG. 8, the second part may be a finger of the user shown in FIG. 6 or FIG. 8, the first physiological parameter may be upper limb impedance, the n second physiological parameters may include one or more of the following parameters: a body fat rate, a visceral fat content in a torso, or a torso fat content, and the third physiological parameter may be a liver fat content or a liver risk level.

In a possible implementation, the method includes: generating the first current; determining the first voltage value between the first group of electrodes and the second group of electrodes; and determining the first physiological parameter based on the first current value and the first voltage value. The method further includes: generating a second current of a first frequency, where the second current has a second current value; determining a second voltage value between the first group of electrodes and the second group of electrodes; generating a third current of a second frequency, where the third current has a third current value; determining a third voltage value between the first group of electrodes and the second group of electrodes; and determining the first physiological parameter based on the second current value, the second voltage value, the third current value, and the third voltage value. In this way, the first physiological parameter can be measured from a plurality of dimensions by using a plurality of currents of different frequencies, thereby improving accuracy of measuring the third physiological parameter.

In a possible implementation, the method further includes: determining whether the first physiological parameter is within a preset range; and if the first physiological parameter is not within the preset range, performing re-measurement as prompted.

In a possible implementation, the method includes: obtaining the third physiological parameter based on the n second physiological parameters, where the obtaining specifically includes: obtaining a fourth physiological parameter; and obtaining the third physiological parameter based on the n second physiological parameters and the fourth physiological parameter. For example, the fourth physiological parameter includes the waist circumference and/or the height. Therefore, the third physiological parameter is obtained with reference to the fourth physiological parameter, to improve accuracy of measuring the third physiological parameter.

In a possible implementation, the obtaining the third physiological parameter based on the n second physiological parameters and the fourth physiological parameter specifically includes: determining a time difference between a currently obtained fourth physiological parameter and a previously obtained fourth physiological parameter; and obtaining the third physiological parameter based on the time difference, the n second physiological parameters, and the currently obtained fourth physiological parameter. For example, the fourth physiological parameter is a waist circumference. In this way, the third physiological parameter is corrected based on the input time difference of the fourth physiological parameter, to improve accuracy of measuring the third physiological parameter.

In a possible implementation, the second group of electrodes are integrated in a physical button on the lateral side of the device body.

In a possible implementation, the first group of electrodes include one electrode, and the second group of electrodes include one electrode; or the first group of electrodes include two electrodes, and the second group of electrodes include two electrodes.

In a possible implementation, the second group of electrodes include a first electrode and a second electrode, and the first electrode and the second electrode are located on a same side of the device body.

According to a third aspect, this application provides a wearable device, including:

a device body, where a display screen, a first group of electrodes, and a second group of electrodes are disposed on the device body, the first group of electrodes are located on a side that is on the device body but is opposite to the display screen, and the second group of electrodes are located on a lateral side of the device body;
a memory, where the memory stores a computer program; and
a processor, where the processor is electrically connected to the first group of electrodes and the second group of electrodes.

When the computer program stored in the memory is executed by the processor, the wearable device is enabled to perform the method according to the first aspect or the second aspect.

In a possible implementation, the second group of electrodes are integrated in a physical button on the lateral side of the device body.

In a possible implementation, the first group of electrodes include one electrode, and the second group of electrodes include one electrode; or the first group of electrodes include two electrodes, and the second group of electrodes include two electrodes.

In a possible implementation, the second group of electrodes include a first electrode and a second electrode, and the first electrode and the second electrode are located on a same side of the device body.

According to a fourth aspect, this application provides a computer storage medium, where the computer storage medium stores instructions, and when the instructions are run on a computer, the computer is enabled to perform the method according to the first aspect or the second aspect.

According to a fifth aspect, this application provides a computer program product that includes instructions. When the instructions are run on a computer, the computer is enabled to perform the method according to the first aspect or the second aspect.

BRIEF DESCRIPTION OF DRAWINGS

The following briefly describes accompanying drawings required in descriptions of embodiments or the prior art.

FIG. 1 is a schematic structural diagram of a wearable device according to an embodiment of this application;

FIG. 2 is a schematic structural diagram of a wearable device according to an embodiment of this application;

FIG. 3 is a schematic structural diagram of a wearable device according to an embodiment of this application;

FIG. 4 is a schematic structural diagram of a wearable device according to an embodiment of this application;

FIG. 5 is a schematic structural diagram of a processor on a wearable device according to an embodiment of this application;

FIG. 6 is a schematic diagram of an electrode placement position on a wearable device according to an embodiment of this application;

FIG. 7 is a schematic diagram of a conduction position when electrodes on a wearable device are conducted according to an embodiment of this application;

FIG. 8 is a schematic diagram of electrode placement positions on a wearable device according to an embodiment of this application;

FIG. 9 is a schematic diagram of equivalent impedance of a human body in a process of measuring upper limb impedance according to an embodiment of this application;

FIG. 10 is a schematic principle diagram of measuring upper limb impedance according to an embodiment of this application;

FIG. 11 is a schematic principle diagram of measuring upper limb impedance according to an embodiment of this application;

FIG. 12 is a schematic diagram of a process of measuring a liver risk level according to an embodiment of this application;

FIG. 13a is a schematic diagram of steps of measuring a liver fat content according to an embodiment of this application;

FIG. 13b is a schematic diagram of steps of measuring a liver fat content according to an embodiment of this application;

FIG. 13c is a schematic diagram of steps of measuring a liver fat content according to an embodiment of this application;

FIG. 14 is a schematic diagram of steps of measuring liver impedance according to an embodiment of this application;

FIG. 15a is a schematic operation diagram of measuring first impedance from the left upper limb to an upper part of the liver according to an embodiment of this application;

FIG. 15b is a schematic operation diagram of measuring second impedance from the left upper limb to a lower part of the liver according to an embodiment of this application;

FIG. 16a is a schematic diagram of contact positions between electrodes on a wearable device and a user body in a process of measuring first impedance from the left upper limb to an upper part of the liver according to an embodiment of this application;

FIG. 16b is a schematic diagram of contact positions between electrodes on a wearable device and a user body in a process of measuring second impedance from the left upper limb to a lower part of the liver according to an embodiment of this application;

FIG. 17 is a schematic diagram of equivalent impedance of a human body in a process of measuring liver impedance according to an embodiment of this application;

FIG. 18 is a schematic diagram of steps of measuring liver impedance according to an embodiment of this application;

FIG. 19a is a schematic operation diagram of measuring upper limb impedance according to an embodiment of this application;

FIG. 19b is a schematic operation diagram of measuring impedance from the left upper limb to the liver according to an embodiment of this application;

FIG. 20 is a schematic diagram of equivalent impedance of a human body in a process of measuring liver impedance according to an embodiment of this application;

FIG. 21 is a schematic diagram of a process of measuring a liver risk level according to an embodiment of this application;

FIG. 22 is a schematic diagram of interface changes on a wearable device in a process of measuring a liver risk level according to an embodiment of this application; and

FIG. 23 is a schematic diagram of a process of measuring a liver risk level according to an embodiment of this application.

## DESCRIPTION OF EMBODIMENTS

To make objectives, technical solutions, and advantages of embodiments of this application clearer, the following describes the technical solutions in embodiments of this application with reference to accompanying drawings.

Terms used in the following embodiments are merely intended to describe specific embodiments, but are not intended to limit this application. The terms "one", "a", and "this" of singular forms used in this specification and the appended claims of this application are also intended to include expressions such as "one or more", unless otherwise specified in the context clearly. It should be further understood that in the following embodiments of this application, "at least one" and "one or more" mean one, two, or more. The term "and/or" is used to describe an association relationship between associated objects and represents that three relationships may exist. For example, A and/or B may represent the following cases: Only A exists, both A and B exist, and only B exists, where A and B each may be singular or plural. The character "/" generally indicates an "or" relationship between the associated objects.

Reference to "an embodiment", "some embodiments", or the like described in this specification indicates that one or more embodiments of this application include a specific feature, structure, or characteristic described with reference to the embodiment. Therefore, statements such as "in an embodiment", "in some embodiments", "in some other embodiments", and "in other embodiments" that appear at different places in this specification do not necessarily mean referring to a same embodiment. Instead, the statements mean "one or more but not all of embodiments", unless otherwise specifically emphasized in another manner. The terms "include", "have", and their variants all mean "include but are not limited to", unless otherwise specifically emphasized in another manner. The term "connection" includes a direct connection and an indirect connection, unless otherwise indicated.

The terms "first" and "second" mentioned below are merely intended for a purpose of description, and shall not be understood as an indication or implication of relative importance or implicit indication of the number of indicated technical features. Therefore, a feature limited by "first" or "second" may explicitly or implicitly include one or more features.

In embodiments of this application, the word "example", "for example", or the like is used to represent giving an example, an illustration, or a description. Any embodiment or design scheme described as "example" or "for example" in embodiments of this application should not be explained as being more preferred or having more advantages than another embodiment or design scheme. Exactly, use of the term "example", "for example", or the like is intended to present a relative concept in a specific manner.

An embodiment of this application provides a wearable device. The wearable device may examine liver fat of a user. For example, the wearable device includes but is not limited to a watch, a band, or the like.

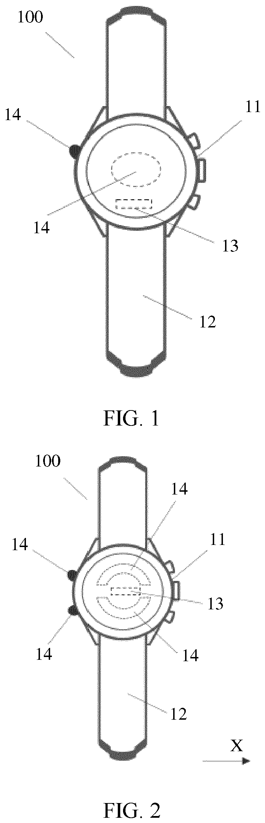

For example, FIG. 1 is a schematic structural diagram of a wearable device according to this embodiment of this application. As shown in FIG. 1, the wearable device 100 includes a device body 11, a flexible fixing band 12 connected to two ends of the device body 11, a processor 13, and at least two groups of electrodes. Each group of electrodes may include at least one electrode 14. The processor 13 is disposed in the device body 11, and each electrode 14 is electrically connected to the processor 13.

Still refer to FIG. 1. A display screen (not shown in the figure) may be disposed on the device body 11. At least one group of electrodes are disposed on a side that is on the device body 11 but is opposite to the display screen (that is, on a rear part of the device body), and at least one group of electrodes are disposed on a lateral side of the device body 11. The lateral side may be understood as a left side or a right side of the device body 11 shown in FIG. 1. In this embodiment, the electrode 14 included in each group of electrodes may be coated on the device body 11, or may be pasted on the device body 11. This is not limited herein. For example, the electrode 14 may be coated or pasted on the rear part, the left side, or the right side of the device body 11. In addition, the electrode 14 included in the at least one group of electrodes located on the lateral side of the device body 11 may be further integrated in a physical button (for example, a power-on button or a function selection button) on the device body 11. For example, a shape of the electrode 14 included in each group of electrodes may be one or more of the following: a circle, an ellipse, a semicircle, an arc, or a rectangle. For example, a size of the electrode 14 may be but is not limited to 8 to 15 square millimeters.

Still refer to FIG. 1. When two groups of electrodes are disposed on the device body 11, and each group of electrodes include one electrode 14, the electrode 14 disposed on the rear part of the device body 11 may be located at a central position on the rear part of the device body 11, or may be located at another position on the rear part of the device body 11, for example, located at a position above the central position of the device body 11. This is not limited herein. The electrode 14 disposed on the left side or the right side of the device body 11 may be located at any position on the left side or the right side of the device body 11. This is not limited herein.

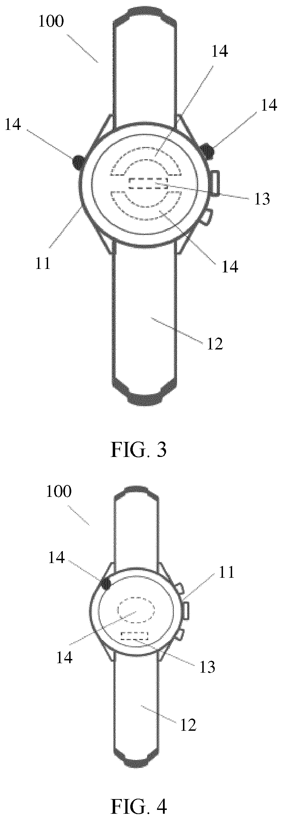

For example, as shown in FIG. 2, when two groups of electrodes are disposed on the device body 11, and each group of electrodes include two electrodes 14, the electrodes 14 disposed on the rear part of the device body 11 may be symmetrically arranged along a center line of a specific direction on the device body 11 (for example, symmetrically arranged along a center line of a direction X shown in the figure), and there is a preset spacing between different electrodes, for example, a spacing of 2 millimeters. This is not limited herein. The two electrodes 14 disposed on the lateral side of the device body 11 may be disposed on a same side, and there is a preset spacing between different electrodes, for example, a spacing of 1 to 2 centimeters. This is not limited herein. In addition, as shown in FIG. 3, two electrodes 14 disposed on the lateral side of the device body 11 may be disposed on different lateral sides of the device body 11. For example, one electrode 14 is disposed on the left side, and the other electrode 14 is disposed on the right side. In addition, as shown in FIG. 4, when two groups of electrodes are disposed on the device body 11, one group of electrodes may be disposed on a rear part of the device body 11, and the other group of electrodes may be disposed on a front part of the device body 11 (that is, a side on which a display screen of the device body 11 is located). The electrode 14 included in the group of electrodes located on the front part of the device body 11 may be randomly arranged. This is not limited herein.

It may be understood that, in FIG. 1 to FIG. 4, the electrode 14 located on the rear part of the device body 11 is represented by a dashed line, which indicates that the electrode 14 is located on the rear part of the device body 11. In other words, FIG. 1 to FIG. 4 show a front view of the wearable device 100, that is, a view of the wearable device 100 from a side on which the display screen of the device body 11 is located. In addition, when three groups of electrodes or more groups of electrodes are disposed on the wearable device 100, for an arrangement manner of the plurality of groups of electrodes on the wearable device 100, refer to the foregoing arrangement manner of the two groups of electrodes. Details are not described herein again.

It may be understood that, a shape and a material of the flexible fixing band 12 are not limited in this embodiment. For example, the flexible fixing band 12 may be implemented by using one flexible band, and two ends of the flexible band are respectively connected to two ends of the device body 11. The flexible fixing band 12 may be further implemented by using two flexible bands. One end of one flexible band is connected to one end of the device body 11, one end of the other flexible band is connected to the other end of the device body 11, and two other ends of the two flexible bands are detachably connected by using a connection structure, such as a clamping or threaded connection. For example, the flexible fixing band 12 may be understood as a band that binds the device body 11 of the wearable device 100 to a user body, so that the user can carry the wearable device 100. For example, when the wearable device 100 is a watch or a band, the flexible fixing band 12 may be referred to as a watch band.

In addition, the processor 13 in this embodiment is a computing core and a control core of the wearable device 100. The processor 13 may include one or more processing units. For example, the processor 13 may include one or more of the following: an application processor (application processor, AP), a modem (modem), a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent components, or may be integrated into one or more processors. In an example, the processor 13 may implement or perform the methods and steps disclosed in embodiments of this application. For example, the processor 13 may control generation of electrical signals of at least one frequency, to determine body impedance (for example, upper limb impedance) of the user by using a plurality of groups of electrodes. The processor 13 may further obtain a liver risk level of the user based on the determined body impedance.

For example, as shown in FIG. 5, a current generation unit 131, a voltage measurement unit 132, and a control and processing unit 133 may be disposed in the processor 13 of the wearable device 100. The current generation unit 131 may generate a current under control of the control and processing unit 133, for example, generate a current of 50 $\mu$A($\mu$A) or 100 $\mu$A($\mu$A). The current generation unit 131 may be connected to an electrode in the wearable device 100. In addition, the current generation unit 131 may also generate currents of different frequencies under control of the control and processing unit 133, for example, generate a current of 50 kilohertz (kHz) or 250 kilohertz (kHz). The voltage measurement unit 132 may measure a voltage. The voltage measurement unit 132 may be connected to an electrode in the wearable device 100. The control and processing unit 133 may control the current generation unit 131 to generate a current, and may further determine impedance of a human body based on a voltage value measured by the voltage measurement unit 132 and a current value generated by the current generation unit 131. For example, the impedance of the human body may be a ratio of the voltage value measured by the voltage measurement unit 132 to the current value generated by the current generation unit 131. For example, current values of currents of different frequencies may be identical, voltage values corresponding to currents of different frequencies may be identical or different, and currents of different frequencies are generated at different moments.

For example, after the user starts measurement, the current generation unit 131 may apply a voltage between one electrode in a group of electrodes and one electrode in another group of electrodes. After the user contacts both electrodes on the wearable device 100 with the body, the two electrodes are conducted, and a current is generated. In this case, the current generation unit 131 may adjust the voltage value, so that the current reaches a preset current value (for example, 50 $\mu$A), and control the current to be continuously maintained at the current value during measurement. For example, when the current value reaches the preset current value, the voltage may be kept unchanged, so as to keep the current value unchanged. For example, after the user starts measurement, the current generation unit 131 may apply a preset voltage value between one electrode in one group of electrodes and one electrode in the other group of electrodes. After the user contacts both electrodes on the wearable device 100 with the body, the two electrodes are conducted, and a current is generated. In this case, the current generation unit 131 may measure the current value. Then, an impedance value may be obtained based on the current value and the preset voltage value. In addition, to improve measurement accuracy, the voltage measurement unit 132 may also measure a voltage value, and determine the impedance value based on the measured voltage value and the measured current value. It may be understood that, in this embodiment, the current generation unit 131, the voltage measurement unit 132, and the control and processing unit 133 are all integrated in the processor 13, or any one or two of the current generation unit 131, the voltage measurement unit 132, and the control and processing unit 133 may be integrated in the processor 13 while the remaining can be disposed separately. In addition, the current generation unit 131, the voltage measurement unit 132, and the control and processing unit 133 may also be separately disposed. This is not limited herein.

The foregoing is a related description of hardware of the wearable device 100 in embodiments of this application. Based on the wearable device 100 described above, the following describes a principle and process of measuring physiological parameters (for example, a liver fat content and a liver risk level) according to embodiments of this application. When the physiological parameters are measured, there may be two cases: the wearable device is worn on a user body and the wearable device is not worn on the user body. The following describes the two cases separately.

(1) When the wearable device is worn on the user body

For example, if two groups of electrodes are disposed on the wearable device 100, each group of electrodes include one electrode, one group of electrodes are disposed on a rear part of the device body 11, and the other group of electrodes are disposed on the left side or the right side of the device body 11 (as shown in FIG. 1), when the user uses the wearable device 100 to measure liver fat, as shown in FIG. 6, the user may wear the wearable device 100 on an arm m (for example, the left arm) of the user, in which case, an electrode b on the device body 11 is in contact with the surface of the arm m, and use a finger on another arm (for example, the right arm) to touch an electrode a on the lateral side of the device body 11. Then, the two groups of electrodes (that is, electrodes a and b) on the wearable device 100 are conducted. In this case, upper limb impedance of the user may be obtained. As shown in FIG. 7, the upper limb impedance may be impedance between the left arm and the right arm of the user, that is, impedance between a point e and a point f in the figure. The point e may be understood as a point at which the electrode b on the rear part of the device body 11 of the wearable device 100 contacts the user body, and the point f may be understood as a point at which the electrode a on the left or right side of the device body 11 of the wearable device 100 contacts the user body (that is, a finger).

If two groups of electrodes are disposed on the wearable device 100, each group of electrodes include two electrodes, one group of electrodes are disposed on a rear part of the device body 11, and the other group of electrodes are disposed on the left side or the right side of the device body 11 (as shown in FIG. 2), when the user uses the wearable device 100 to measure liver fat, as shown in FIG. 8, the user may wear the wearable device 100 on an arm m (for example, the left arm) of the user, in which case, electrodes b and d on the device body 11 are in contact with the surface of the arm m, and use two fingers on another arm (for example, the right arm) to touch electrodes a and c on the lateral side of the device body 11. Then, the two groups of electrodes on the wearable device 100 are conducted (that is, the electrodes a and b are conducted, and the electrodes c and d are conducted). In this case, upper limb impedance of the user may be obtained. Still refer to FIG. 7. The upper limb impedance may be impedance between the left arm and the right arm of the user, that is, impedance between a point e and a point f in the figure. The point e may be understood as a point at which an electrode on the rear part of the device body 11 of the wearable device 100 contacts the user body, and the point f may be understood as a point at which an electrode on the left or right side of the device body 11 of the wearable device 100 contacts the user body (that is, a finger).

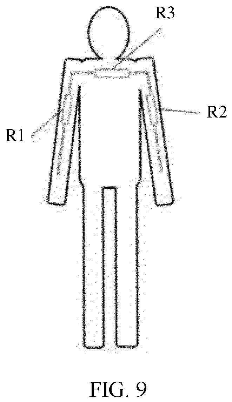

In a process of obtaining the upper limb impedance, as shown in FIG. 6 and FIG. 8, the left arm of the user is equivalent to a resistor, the right arm of the user is equivalent to a resistor, and the chest of the user is equivalent to a resistor. For example, as shown in FIG. 9, the left arm of the user is equivalent to impedance R1, the right arm of the user is equivalent to impedance R2, and the chest of the user is equivalent to impedance R3. Upon measurement according to FIG. 6 and FIG. 8, the upper limb impedance R = R1 + R2 + R3. In an example, an arm of the user shown in FIG. 6 or FIG. 8 may also be referred to as a first part, a finger of the user shown in FIG. 6 or FIG. 8 may also be referred to as a second part, and the upper limb impedance may also be referred to as a first physiological parameter.

For ease of understanding a principle of measuring the upper limb impedance, the following uses an example for description.

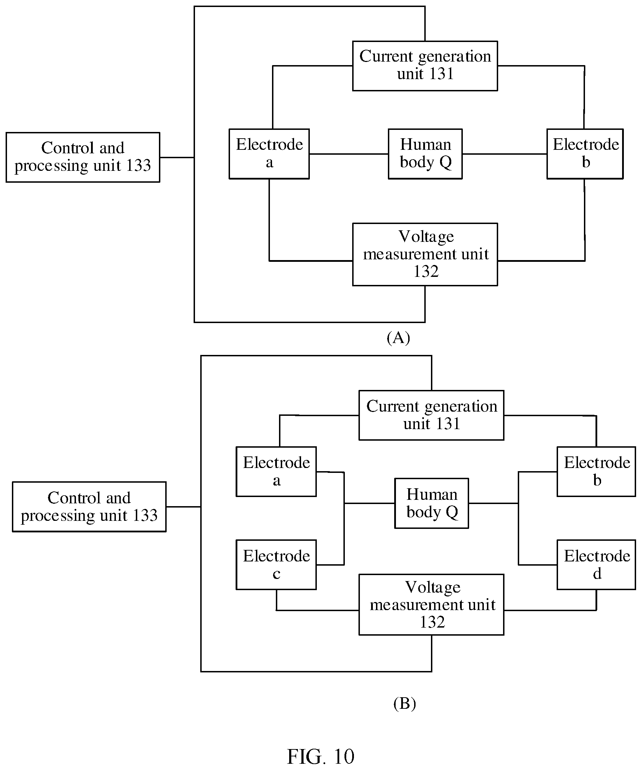

For example, when the upper limb impedance is measured by using two electrodes (a case shown in FIG. 6), as shown in (A) in FIG. 10, a current loop may be formed between the electrode a, the current generation unit 131, and the electrode b on the wearable device 100 and the human body Q. Furthermore, a voltage measurement loop may be formed between the electrode a, the voltage measurement unit 132, and the electrode b on the wearable device 100 and the human body Q. During measurement, the control and processing unit 133 in the processor 13 of the wearable device 100 may control the current generation unit 131 to generate a current I, and the current I flows through the formed current loop. In addition, the voltage measurement unit 132 may measure a voltage U (that is, a voltage of the human body) between the electrodes a and b. Finally, the impedance of the human body Q is obtained based on the voltage U and the current I.

When the upper limb impedance is measured by using four electrodes (a case shown in FIG. 8), as shown in (B) in FIG. 10, a current loop may be formed between the electrode a, the current generation unit 131, and the electrode b on the wearable device 100 and the human body Q. Furthermore, a voltage measurement loop may be formed between the electrode c, the voltage measurement unit 132, and the electrode d on the wearable device 100 and the human body Q. During measurement, the control and processing unit 133 in the processor 13 of the wearable device 100 may control the current generation unit 131 to generate a current I, and the current I flows through the formed current loop. In addition, the voltage measurement unit 132 may measure a voltage U (that is, a voltage of the human body) between the electrodes c and d. Finally, the impedance of the human body Q is obtained based on the voltage U and the current I. It may be understood that, when more electrodes are used to measure the upper limb impedance, reference may be made to a measurement method using two or four electrodes, and details are not described herein again.

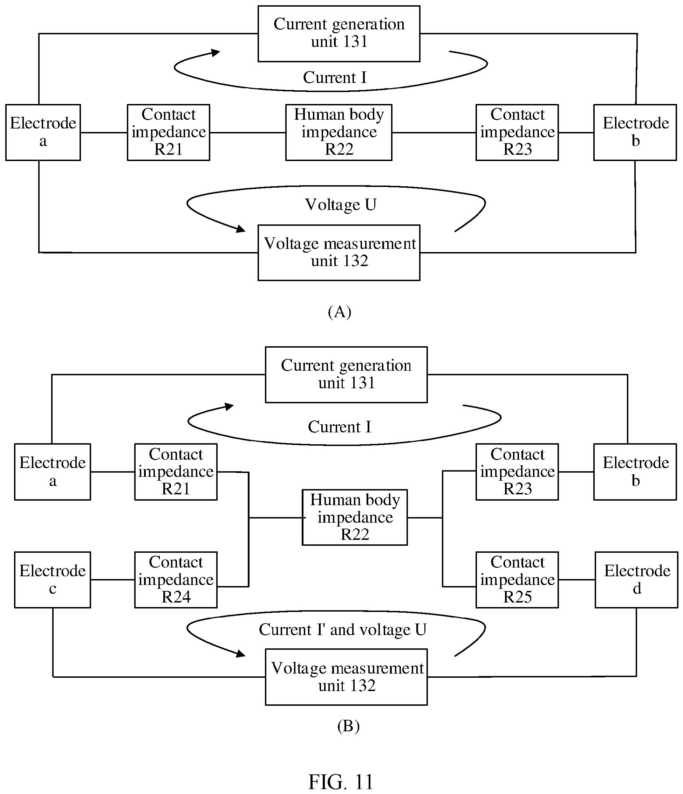

It may be understood that when an electrode on the wearable device 100 is in contact with the user body, there is contact impedance between the electrode and the user body. When the upper limb impedance is measured by using two electrodes (a case shown in FIG. 6), as shown in (A) in FIG. 11, if a current generated by the current generation unit 131 is I, the contact impedance is R21 and R23, and human body impedance is R22, then a voltage measured by the voltage measurement unit 133 is U = I × R21 + I × R22 + I × R23. Therefore, measured impedance is actually U/I = R21 + R22 + R23. In this case, the obtained impedance is the actual human body impedance plus two contact impedance values. Generally, the human body impedance is about 300 S2 to 1,000 Ω, and the contact impedance may be greater than 30 Q. Therefore, the contact impedance has a great impact on a measurement result. In other words, precision of the upper limb impedance measured by using two electrodes is relatively low.

When the upper limb impedance is measured by using four electrodes (a case shown in FIG. 8), as shown in (B) in FIG. 11, if a current generated by the current generation unit 131 is I, the contact impedance is R21, R23, R24, and R25, and the human body impedance is R22, where the human body impedance R22 and contact impedance R24 and contact impedance R25 are connected in series in the voltage measurement loop; and when measuring a voltage, the voltage measurement unit 132 may generate a current I' approximate to 0, in which case, a current flowing through the human body is I + I', then a voltage measured by the voltage measurement unit 132 is U = I' × R24 + (I + I') × R22 + I' × R25. Because I' is approximately 0, U = I × R22. Therefore, the obtained human body impedance is U/I = R22. In this case, the obtained human body impedance may reflect the actual human body impedance. In other words, precision of the upper limb impedance measured by using four electrodes is relatively high.

After the upper limb impedance is measured, another physiological parameter of the user, such as a body fat rate, a visceral fat content in a torso, or a torso fat content, may be obtained based on the upper limb impedance. In an example, a physiological parameter, such as a body fat rate, a visceral fat content in a torso, or a torso fat content, may also be referred to as a second physiological parameter.

For example, the body fat rate may be calculated by using the following formula. The formula (referred to as "Formula 1" below) is:

$$\mathrm{BFR} = \alpha_1 R + \alpha_2 \quad \text{Formula 1}$$

BFR is the body fat rate, R is the upper limb impedance, and $\alpha_1$. and $\alpha_2$ are preset coefficients, which may be obtained by experiment.

In addition, when the upper limb impedance is measured under a plurality of currents of different frequencies, the body fat rate may be calculated by using the following formula. The formula (referred to as "Formula 2" below) is:

$$\mathrm{BFR} = \alpha_1 R_1 + \alpha_2 R_2 + \alpha_3 \quad \text{Formula 2}$$

BFR is the body fat rate, $R_1$ and $R_2$ are the upper limb impedance measured under currents of different frequencies, and $\alpha_1$, $\alpha_2$, and $\alpha_3$ are preset coefficients, which may be obtained by experiment. It may be understood that, when the number of upper limb impedance values measured under currents of different frequencies is greater than or equal to 3, "$a_n R_n$" may be added to Formula 2, where $R_n$ is upper limb impedance measured under a current of an $n^{th}$ frequency, and $\alpha_n$, which may be obtained by experiment, is a preset coefficient corresponding to $R_n$.

The visceral fat content in the torso can be calculated by using the following formula. The formula (referred to as "Formula 3" below) is:

$$\mathrm{X} = \beta_1 R + \beta_2 \quad \text{Formula 3}$$

X is the visceral fat content in the torso, R is the upper limb impedance, and $\beta_1$ and $\beta_2$ are preset coefficients, which may be obtained by experiment.

In addition, when the upper limb impedance is measured under a plurality of currents of different frequencies, the visceral fat content in the torso may be calculated by using the following formula. The formula (referred to as "Formula 4" below) is:

$$X = \beta_1 R_1 + \beta_2 R_2 + \beta_3 \quad \text{Formula 4}$$

X is the visceral fat content in the torso, $R_1$ and $R_2$ are the upper limb impedance measured under currents of different frequencies, and $\beta_1$, $\beta_2$, and $\beta_3$ are preset coefficients, which may be obtained by experiment. It may be understood that, when the number of upper limb impedance values measured under currents of different frequencies is greater than or equal to 3, " $\beta_n R_n$" may be added to Formula 4, where $R_n$ is upper limb impedance measured under a current of an $n^{th}$ frequency, and $\beta_n$, which may be obtained by experiment, is a preset coefficient corresponding to $R_n$.
The torso fat content may be calculated by using the following formula. The formula (referred to as "Formula 5" below) is:

$$P = \theta_1 R + \theta_2 \quad \text{Formula 5}$$

P is the torso fat content, R is the upper limb impedance, and $\theta_1$ and $\theta_2$ are preset coefficients, which may be obtained by experiment.
In addition, when the upper limb impedance is measured under a plurality of currents of different frequencies, the torso fat content may be calculated by using the following formula. The formula (referred to as "Formula 6" below) is:

$$P = \theta_1 R_1 + \theta_2 R_2 + \theta_3 \quad \text{Formula 6}$$

P is the torso fat content, $R_1$ and $R_2$ are the upper limb impedance measured under currents of different frequencies, and $\theta_1$, $\theta_2$, and $\theta_3$ are preset coefficients, which may be obtained by experiment. It may be understood that, when the number of upper limb impedance values measured under currents of different frequencies is greater than or equal to 3, "$\theta_n R_n$" may be added to Formula 6, where $R_n$ is upper limb impedance measured under a current of an $n^{th}$ frequency, and $\theta_n$, which may be obtained by experiment, is a preset coefficient corresponding to $R_n$.
For example, when a physiological parameter, such as the body fat rate, the visceral fat content in the torso, or the torso fat content, is obtained based on the upper limb impedance, the physiological parameter may also be determined with reference to body parameters such as the height of the user.
In this case, with reference to the height of the user, the body fat rate may be calculated by using the following formula. The formula (referred to as "Formula 7" below) is:

$$BFR = \alpha_1 R + \alpha_2 H + \alpha_3 \quad \text{Formula 7}$$

BFR is the body fat rate, R is the upper limb impedance, H is the height, and $\alpha_1$, $\alpha_2$, and $\alpha_3$ are preset coefficients, which may be obtained by experiment.
In addition, when the upper limb impedance is measured under a plurality of currents of different frequencies, with reference to the height of the user, the body fat rate may be calculated by using the following formula. The formula (referred to as "Formula 8" below) is:

$$BFR = \alpha_1 R_1 + \alpha_2 R_2 + \alpha_3 H + \alpha_4 \quad \text{Formula 8}$$

BFR is the body fat rate, $R_1$ and $R_2$ are the upper limb impedance measured under currents of different frequencies, and $\alpha_1$, $\alpha_2$, $\alpha_3$, and $\alpha_4$ are preset coefficients, which may be obtained by experiment. It may be understood that, when the number of upper limb impedance values measured under currents of different frequencies is greater than or equal to 3, "$a_n R_n$" may be added to Formula 2, where $R_n$ is upper limb impedance measured under a current of an $n^{th}$ frequency, and $a_n$, which may be obtained by experiment, is a preset coefficient corresponding to $R_n$.
With reference to the height of the user, the visceral fat content in the torso can be calculated by using the following formula. The formula (referred to as "Formula 9" below) is:

$$X = \beta_1 R + \beta_2 H + \beta_3 \quad \text{Formula 9}$$

X is the visceral fat content in the torso, R is the upper limb impedance, and $\beta_1$, $\beta_2$, and $\beta_3$ are preset coefficients, which may be obtained by experiment.

In addition, when the upper limb impedance is measured under a plurality of currents of different frequencies, with reference to the height of the user, the visceral fat content in the torso may be calculated by using the following formula. The formula (referred to as "Formula 10" below) is:

$$X = \beta_1 R_1 + \beta_2 R_2 + \beta_3 H + \beta_4 \quad \text{Formula 10}$$

X is the visceral fat content in the torso, $R_1$ and $R_2$ are the upper limb impedance measured under currents of different frequencies, and $\beta_1$, $\beta_2$, $\beta_3$, and $\beta_4$ are preset coefficients, which may be obtained by experiment. It may be understood that, when the number of upper limb impedance values measured under currents of different frequencies is greater than or equal to 3, "$\beta_n R_n$" may be added to Formula 4, where $R_n$ is upper limb impedance measured under a current of an $n^{th}$ frequency, and $\beta_n$ which may be obtained by experiment, is a preset coefficient corresponding to $R_n$.

With reference to the height of the user, the torso fat content may be calculated by using the following formula. The formula (referred to as "Formula 11" below) is:

$$P = \theta_1 R + \theta_2 H + \theta_3 \quad \text{Formula 11}$$

P is the torso fat content, R is the upper limb impedance, and $\theta_1$, $\theta_2$, and $\theta_3$ are preset coefficients, which may be obtained by experiment.

In addition, when the upper limb impedance is measured under a plurality of currents of different frequencies, with reference to the height of the user, the torso fat content may be calculated by using the following formula. The formula (referred to as "Formula 12" below) is:

$$P = \theta_1 R_1 + \theta_2 R_2 + \theta_3 H + \theta_4 \quad \text{Formula 12}$$

P is the torso fat content, $R_1$ and $R_2$ are the upper limb impedance measured under currents of different frequencies, and $\theta_1$, $\theta_2$, $\theta_3$, and $\theta_4$ are preset coefficients, which may be obtained by experiment. It may be understood that, when the number of upper limb impedance values measured under currents of different frequencies is greater than or equal to 3, "$\theta_n R_n$" may be added to Formula 6, where $R_n$ is upper limb impedance measured under a current of an $n^{th}$ frequency, and $\theta_n$, which may be obtained by experiment, is a preset coefficient corresponding to $R_n$.

Further, after the physiological parameters, such as the body fat rate, the visceral fat content in the torso, and the torso fat content, are obtained, the liver fat content of the user may be determined with reference to a body mass index (body mass index, BMI) of the user. In a formula $BMI = {}^{W}/_{H^2}$, W is a weight, and H is a height.

For example, the liver fat content of the user may be calculated by using the following formula. The formula (referred to as "Formula 13" below) is:

$$M = \gamma_1 BMI + \gamma_2 BFR + \gamma_3 X + \gamma_4 P + \gamma_5 \quad \text{Formula 13}$$

M is the liver fat content, BMI is the body mass index, BFR is the body fat rate, X is the visceral fat content in the torso, P is the torso fat content, and $\gamma_1$, $\gamma_2$, $\gamma_3$, $\gamma_4$, and $\gamma_5$ are preset coefficients, which may be obtained by experiment. It may be understood that parameters (for example, BMI, BFR, X, and P) in Formula 13 may be selected as required. This is not limited herein.

In addition, the liver fat content of the user may also be calculated with reference to a waist circumference of the user. In this case, the liver fat content of the user may be calculated by using the following formula. The formula (referred to as "Formula 14" below) is:

$$M = \gamma_1 BMI + \gamma_2 BFR + \gamma_3 X + \gamma_4 P + \gamma_5 L + \frac{\gamma_6 L}{H} + \gamma_7 \Delta T + \gamma_8 \quad \text{Formula 14}$$

M is the liver fat content, BMI is the body mass index, BFR is the body fat rate, X is the visceral fat content in the torso, P is the torso fat content, L is the waist circumference, H is the height, and $\Delta T$ is a time difference between a moment when a waist circumference is used currently and a moment when a waist circumference is used previously, where the waist circumference used currently is different from the waist circumference used previously. If the waist circumference used currently is the same as the waist circumference used previously, or the time difference between moments when two waist circumferences are used exceeds a preset time threshold, or the waist circumference is used for the first time, $\Delta T$ may be 0. $\gamma_1$, $\gamma_2$, $\gamma_3$, $\gamma_4$, $\gamma_5$, $\gamma_6$, $\gamma_7$, and $\gamma_8$ are preset coefficients, which may be obtained by experiment. It may be understood that parameters (for example, BMI, BFR, X, P, L, H, and $\Delta T$) in Formula 14 may be selected as required. This is not limited herein. In an example, the waist circumference and/or the height may also be referred to as a fourth physiological parameter.

After the liver fat content is obtained, a liver risk level of the user may be determined based on a correspondence between the liver fat content and the liver risk level. For example, a preset correspondence between the liver fat content and the liver risk level may be shown in Table 1. When the liver fat content is determined to be "5", it can be learned from Table 1 that the liver risk level is "Suspected". In an example, the liver fat content or the liver risk level may also be referred to as a third physiological parameter.

**Table 1**

| Liver fat content | Liver risk level |
| --- | --- |
| 0-4 | Normal |
| 4-7 | Suspected |
| 7-10 | Medium to high |

The foregoing is a description of a related measurement principle when the wearable device 100 in the technical solution of this application is worn on the user body. The following uses the case shown in FIG. 2 as an example to describe a process of measuring the liver risk level.

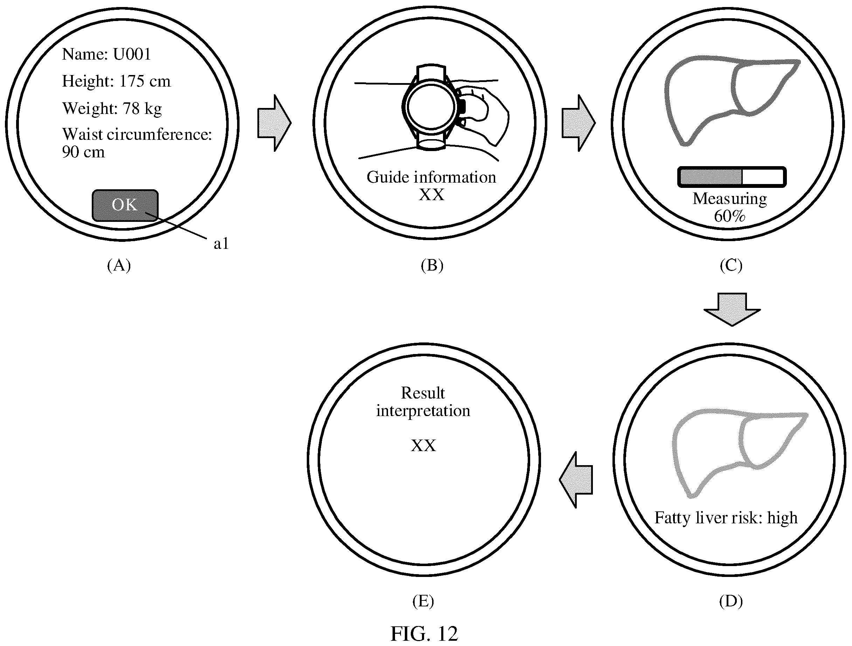

For example, an application program (such as Huawei Health) related to liver fat measurement may be installed on the wearable device 100. As shown in (A) in FIG. 12, after the user opens the application program related to liver fat measurement on the wearable device 100 and starts the liver fat measurement, the wearable device 100 may display personal information of the user, such as the name, the height, the weight, and the waist circumference, where the personal information can be modified by the user. In (A) in FIG. 12, the user may tap an "OK" button in an area a1 to proceed to a next step. Then, as shown in (B) in FIG. 12, the wearable device 100 may display guide information. The guide information is mainly used to guide the user to place an electrode on the wearable device 100 at a specific position of the user body, for example, guide the user to place an electrode on a rear part of the device body 11 of the wearable device 100 on an arm of the user, and place an electrode on the left side or the right side of the device body 11 on a finger of the user. The guide information may be text information, voice information, graphic information, or a combination of any two or three of the text information, the voice information, and the graphic information. It may be understood that, when selecting a button on the wearable device 100, the user may select by tapping, by using voice, or by using a gesture. A specific choice is subject to an actual need. This is not limited herein. In addition, after displaying the guide information (that is, displaying an interface shown in (B) in FIG. 12), the wearable device 100 may apply a current required for measurement. Moreover, before displaying the guide information, the wearable device 100 may also generate a current required for measurement. This is not limited herein.

After the user touches the electrode on the wearable device 100 based on the guide information shown in (B) in FIG. 12, the measurement may be performed. During the measurement, the wearable device 100 may display an interface shown in (C) in FIG. 12. Then, after measuring the liver fat content of the user, the wearable device 100 may prompt, by using voice or by using both voice and text, the user that the measurement is completed, and display a measurement result, that is, display an interface shown in (D) in FIG. 12. In addition, after displaying the interface shown in (D) in FIG. 12, the wearable device 100 may also display related result interpretation, that is, display an interface shown in (E) in FIG. 12. For example, if the risk of fatty liver is high, the result can be interpreted as follows: You have a high risk of fatty liver. For the sake of your health, please strictly control your diet, especially your oil and alcohol intake, and it is recommended that you take aerobic exercise for at least one hour every day. If the risk of fatty liver is moderate, the result can be interpreted as follows: You have a moderate risk of fatty liver. For the sake of your health, please control your diet, especially your oil and alcohol intake, and it is recommended that you take aerobic exercise every day. If the risk of fatty liver is low, the result can be interpreted as follows: You have a low risk of fatty liver. For the sake of your health, please eat a balanced diet, exercise properly, and control your alcohol intake. In addition to displaying the measurement result, the wearable device 100 may also report the measurement result by using voice. In addition to an automatic switch from (D) to (E) in FIG. 12, the user may also switch manually. For example, in (D) in FIG. 12, when the user performs a sliding operation (for example, slides up, slides down, slides left, or slides right) in a direction on the device body 11 of the wearable device 100, the wearable device 100 switches to (E) in FIG. 12.

It may be understood that, in the interface shown in (C) in FIG. 12, before obtaining a liver risk level, the wearable device 100 needs to measure upper limb impedance and then obtain a liver fat content based on the upper limb impedance. When the upper limb impedance is measured, the upper limb impedance may be compared with a preset impedance range to determine whether the measured upper limb impedance is normal. When the upper limb impedance falls within the preset impedance range, the measured upper limb impedance is normal; otherwise, the measured upper limb impedance is abnormal. Further, if it is determined that the measured upper limb impedance is abnormal, the user may be prompted to measure again. For example, the user may be prompted by using voice to measure again. After prompting the user, the wearable device 100 may return to the interface shown in (B) in FIG. 12.

In addition, if the user does not enter information such as "height", "weight", and "waist circumference" on an interface shown in (A) in FIG. 12, or the wearable device 100 does not obtain information such as "height", "weight", and "waist circumference" from information stored in the wearable device 100 or from another electronic device, on the interface shown in (C) in FIG. 12, the liver fat content may be calculated by using "Formula 13" described above. In this case, the user can choose not to use the parameter "BMI".

If the user does not enter the "waist circumference" on the interface shown in (A) in FIG. 12, or the wearable device 100 does not obtain information such as the "waist circumference" from information stored in the wearable device 100 or from another electronic device, on the interface shown in (C) in FIG. 12, the liver fat content may be calculated by using "Formula 13" described above. If the user inputs the "waist circumference" on the interface shown in (A) in FIG. 12, on the interface shown in (C) in FIG. 12, the liver fat content may be calculated by using "Formula 14" described above. In this case, in the absence of information such as "height" and/or "weight", the user may choose not to use the parameter "BMI".

For ease of understanding different calculation processes under a circumstance where the "waist circumference" is entered and another circumstance where the "waist circumference" is not entered, the following uses an example for description. For example, selection of a calculation model (that is, a calculation formula) involved when the "waist circumference" is entered and when the "waist circumference" is not entered may include three cases. In a first case, a proper calculation model is selected for calculation based on a case whether the waist circumference is entered. In a second case, a calculation model is used for calculation regardless of whether the waist circumference is entered. In a third case, a calculation model can be used for calculation only when the waist circumference is entered. The following describes the three cases separately.

(a) First case

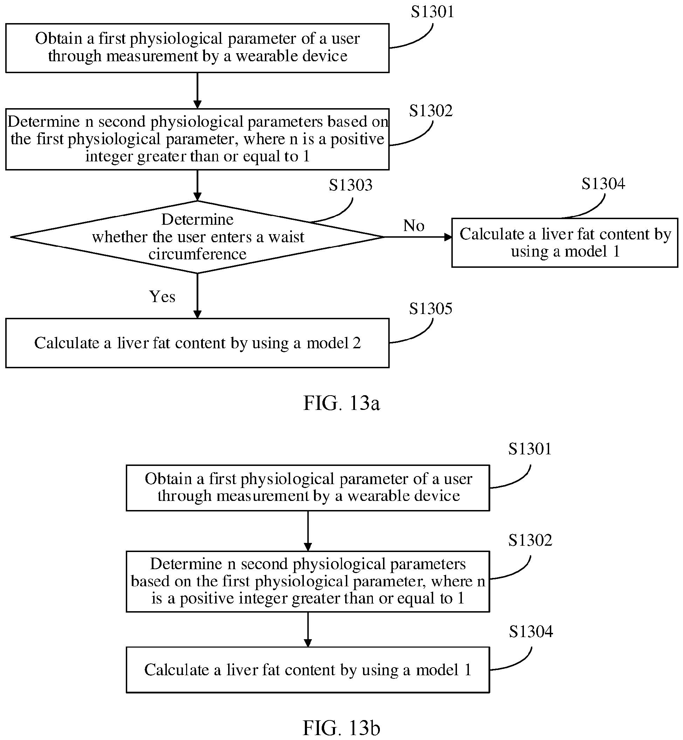

FIG. 13a is a schematic diagram of steps of measuring a liver fat content. Specifically, as shown in FIG. 13a, the following steps are included:

S1301: Obtain a first physiological parameter of the user through measurement by the wearable device 100.

For example, the first physiological parameter may be upper limb impedance. The first physiological parameter may be obtained by using the wearable device 100 in a measurement manner shown in FIG. 6 or FIG. 8.

S1302: Determine n second physiological parameters based on the first physiological parameter, where n is a positive integer greater than or equal to 1.

For example, the first physiological parameter may be the upper limb impedance, and the n second physiological parameters may include one or more of the following parameters: a body fat rate, a visceral fat content in a torso, or a torso fat content. The n second physiological parameters may be determined by using "Formula 1 to Formula 12" described above.

S1303: Determine whether the user enters a waist circumference.

For example, during measurement, when the user enters the waist circumference on the interface shown in (A) in FIG. 12 for measurement, the wearable device 100 may determine, based on information entered by the user, whether the user enters the waist circumference. If the user does not enter the waist circumference, S1304 is performed, that is, the liver fat content is determined by using a model 1 (that is, "Formula 13" described above). If the user enters the waist circumference, S1305 is performed, that is, the liver fat content is determined by using a model 2 (that is, "Formula 14" described above).

S1304: Calculate the liver fat content by using the model 1.

Specifically, if the user does not enter the waist circumference, the liver fat content is determined by using the model 1 (that is, "Formula 13" described above).

S1305: Calculate the liver fat content by using the model 2.

Specifically, if the user enters the waist circumference, the liver fat content is determined by using the model 2 (that is, "Formula 14" described above).

It may be understood that, in the first case, a proper calculation model may be selected for calculation based on a case whether the user enters the waist circumference.

(b) Second case

FIG. 13b is a schematic diagram of steps of measuring a liver fat content. Specifically, as shown in FIG. 13b, the following steps are included:

S1301: Obtain a first physiological parameter of the user through measurement by the wearable device 100.
S1302: Determine n second physiological parameters based on the first physiological parameter, where n is a positive integer greater than or equal to 1.
S1304: Calculate the liver fat content by using a model 1.

It may be understood that, in the second case, regardless of whether the user enters a "waist circumference", the liver fat content is determined by using the model 1 (that is, "Formula 13" described above).

(c) Third case

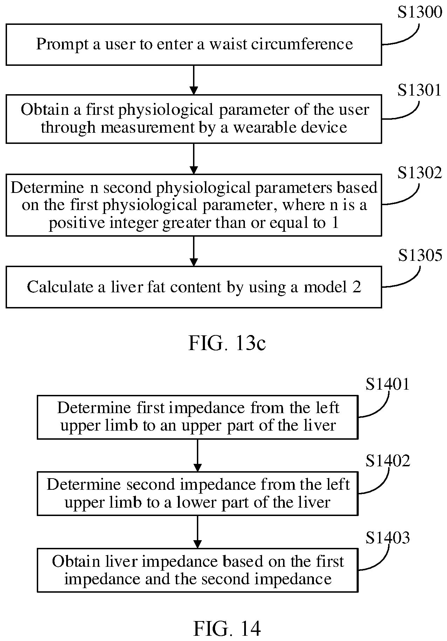

FIG. 13c is a schematic diagram of steps of measuring a liver fat content. Specifically, as shown in FIG. 13c, the following steps are included:
S1300: Prompt the user to enter a waist circumference.
Specifically, in the third case, to perform calculation, the user needs to enter the "waist circumference". In this case, the user may be required to enter the "waist circumference". For example, still refer to (A) in FIG. 12. On the display interface, only after entering the "waist circumference", the user can tap an "OK" button in an area a1 to proceed to a next step. If the user does not enter the "waist circumference", the "OK" button in the area a1 is untappable.
S1301: Obtain a first physiological parameter of the user through measurement by the wearable device 100.
S1302: Determine n second physiological parameters based on the first physiological parameter, where n is a positive integer greater than or equal to 1.
S1305: Calculate the liver fat content by using a model 2.
It may be understood that, in the third case, to perform calculation, the user needs to enter the "waist circumference", and the liver fat content is determined by using the model 2 (that is, "Formula 14" described above). In addition, an execution sequence of S1300 is not limited, and S1300 may precede S1305. For example, still refer to FIG. 12. S1300 may be performed before (C) in FIG. 12. Specifically, S1300 may be performed on the interface shown in (A) in FIG. 12, or may be performed between the interface shown in (A) in FIG. 12 and the interface shown in (C) in FIG. 12. For example, if the user does not enter the "waist circumference", the wearable device 100 may display, between (A) and (B) in FIG. 12, an interface prompting the user to enter the waist circumference, or may display, between (B) and (C) in FIG. 12, an interface prompting the user to enter the waist circumference. This is not limited herein.
It should be noted that the display interfaces shown in FIG. 12 are merely examples for description, and do not constitute a limitation on this solution. The display interfaces may be selected or re-drawn as required. This is not limited herein.
It may be understood that information such as "weight" and "waist circumference" shown in (A) in FIG. 12 may be entered by the user, or may be obtained by the wearable device 100 from another electronic device. For example, the weight information may be obtained from another electronic device (for example, a weight scale) with a weighing function, and the waist circumference information may be obtained from another electronic device with a waist circumference measurement function. In an example, the wearable device 100 may perform short-range communication or long-range communication with another electronic device, to implement information exchange between the wearable device 100 and the another electronic device. For example, a communication module may be disposed in the wearable device 100. The communication module may include at least one filter, a switch, a power amplifier, a low noise amplifier (low noise amplifier, LNA), and the like. The communication module may receive an electromagnetic wave by using at least one antenna, perform processing such as filtering and amplification on the received electromagnetic wave, and transmit the processed electromagnetic wave to a modem for demodulation. The communication module may further amplify a signal modulated by the modem, and convert the signal into an electromagnetic wave through an antenna and radiate the electromagnetic wave. In some examples, at least some function modules of the communication module may be disposed in the processor 13 of the wearable device 100. In some examples, at least some function modules of the communication module and at least some modules of the processor 13 of the wearable device 100 may be disposed in a same component. When the communication module includes a wireless communication module, the communication module may provide a wireless communication solution that is applied to the wearable device 100 and that includes a wireless local area network (wireless local area network, WLAN) (such as a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, and the like. The communication module may be one or more components integrating at least one communication processing module. The communication module receives an electromagnetic wave by an antenna, performs frequency modulation and filtering processing on an electromagnetic wave signal, and sends the processed signal to the processor 13 of the wearable device 100. The communication module may further receive a to-be-sent signal from the processor 13 of the wearable device 100, perform frequency modulation and amplification on the to-be-sent signal, and convert the to-be-sent signal into an electromagnetic wave by using an antenna and radiate the electromagnetic wave.

(2) When the wearable device is not worn on the user body

It may be understood that, in this case, the wearable device may measure liver impedance of the user, so as to obtain a liver fat content of the user based on the measured liver impedance, and further obtain a liver risk level.

The following describes a liver impedance measurement solution provided in an embodiment of this application. For example, as shown in FIG. 14, a method for measuring the liver impedance may include the following steps: S1401: Determine first impedance from the left upper limb to an upper part of the liver.

Specifically, when determining the first impedance from the left upper limb to the upper part of the liver, the user may contact some electrodes on the wearable device 100 with the left hand, and contact the other electrodes on the wearable device 100 with an area in the upper part of the liver on the user body, so as to obtain the first impedance from the left upper limb to the upper part of the liver.

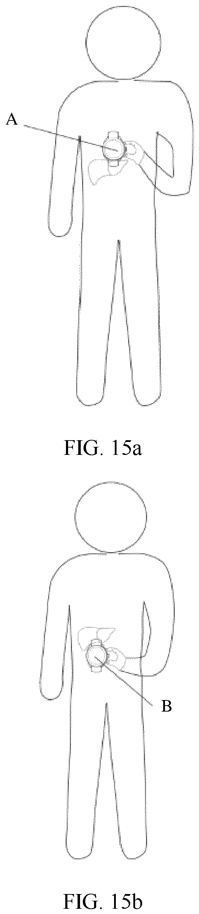

For example, when two groups of electrodes are disposed on the wearable device 100, one group of electrodes are disposed on a rear part of the device body 11, and the other group of electrodes are disposed on the left side or the right side of the device body 11 (as shown in FIG. 1, FIG. 2, FIG. 3, or FIG. 4), the user may place one group of electrodes on the wearable device 100 onto an area in which one end of the liver in the body is located, and touch the other group of electrodes on the wearable device 100 with a finger of the left hand. For example, as shown in FIG. 15a, the user may attach a group of electrodes on the device body 11 of the wearable device 100 onto a xiphoid process of the user body (for example, an area A in the figure), and touch the other group of electrodes on the device body 11 of the wearable device 100 with a finger of the left hand. As shown in FIG. 16a, if an area in which the other group of electrodes on the device body 11 are in contact with the finger of the left hand of the user is an area C in the figure, after the user places the wearable device 100 onto the area A on the user body, and touches the electrodes on the wearable device 100 with the finger, an electrode in contact with the area A on the user body and an electrode in contact with the area C on the user body are conducted. In this case, a current generated by the current generation unit 132 in the processor 13 of the wearable device 100 flows in a loop formed between the areas A and C of the user body. Then, the voltage measurement unit 132 in the processor 13 of the wearable device 100 may measure a voltage value between the two groups of electrodes. Finally, the control and processing unit 133 in the processor 13 of the wearable device 100 may obtain impedance $R_{A-C}$ between the area A and the area C based on the voltage value measured by the voltage measurement unit 132 and a current value of the current generated by the current generation unit 131, that is, obtain the first impedance from the left upper limb to the upper part of the liver. In an example, the area C in FIG. 16a may also be referred to as a first part, the area A in FIG. 16a may also be referred to as a second part, and the first impedance may also be referred to as a first physiological parameter.

S1402: Determine second impedance from the left upper limb to a lower part of the liver.

Specifically, when determining the second impedance from the left upper limb to the lower part of the liver, the user may contact some electrodes on the wearable device 100 with the left hand, and contact the other electrodes on the wearable device 100 with an area in the lower part of the liver on the user body, so as to obtain the second impedance from the left upper limb to the lower part of the liver.

For example, when two groups of electrodes are disposed on the wearable device 100, one group of electrodes are disposed on a rear part of the device body 11, and the other group of electrodes are disposed on the left side or the right side of the device body 11 (as shown in FIG. 1, FIG. 2, FIG. 3, or FIG. 4), the user may place one group of electrodes on the wearable device 100 onto an area in which one end of the liver in the body is located, and touch the other group of electrodes on the wearable device 100 with a finger. For example, as shown in FIG. 15b, the user may attach a group of electrodes on the device body 11 of the wearable device 100 below a rib on a right side of the user body (for example, an area B in the figure) and touch the other group of electrodes on the device body 11 of the wearable device 100 with a finger of the left hand. As shown in FIG. 16b, if an area in which the other group of electrodes on the device body 11 are in contact with the finger of the left hand of the user is an area C in the figure, after the user places the wearable device 100 onto the area B on the user body, and touches the electrodes on the wearable device 100 with the finger, an electrode in contact with the area B on the user body and an electrode in contact with the area C on the user body are conducted. In this case, a current generated by the current generation unit 132 in the processor 13 of the wearable device 100 flows in a loop formed between the areas B and C of the user body. Then, the voltage measurement unit 132 in the processor 13 of the wearable device 100 may measure a voltage value between the two groups of electrodes. Finally, the control and processing unit 133 in the processor 13 of the wearable device 100 may obtain impedance $R_{B-C}$

between the area B and the area C based on the voltage value measured by the voltage measurement unit 132 and a current value of the current generated by the current generation unit 131, that is, obtain the second impedance from the left upper limb to the lower part of the liver. In an example, the area B in FIG. 16b may also be referred to as a third part, and the second impedance may also be referred to as a second physiological parameter.

S1403: Obtain liver impedance based on the first impedance and the second impedance.

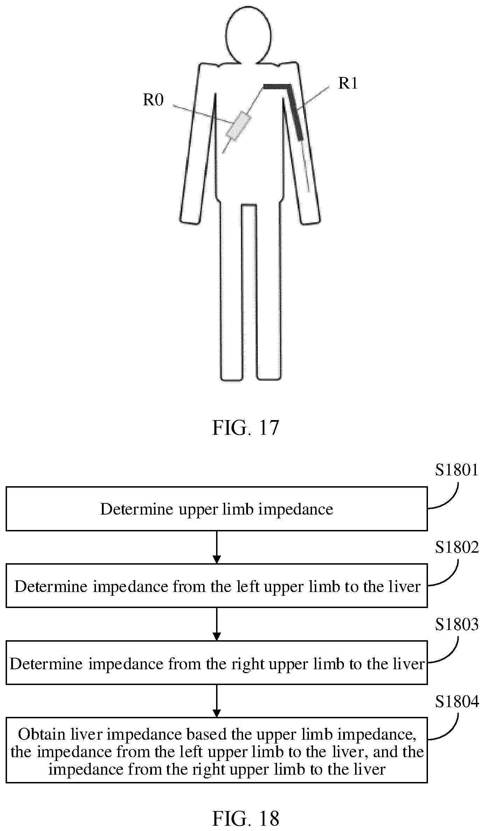

Specifically, after the first impedance and the second impedance are obtained, the liver impedance may be obtained based on the first impedance and the second impedance. In a process of obtaining the liver impedance, the chest and an arm of the user is equivalent to a resistor, and the liver of the user is equivalent to a resistor. For example, as shown in FIG. 17, the liver of the user is equivalent to impedance $R_0$, and the chest and the left arm of the user is equivalent to impedance $R_1$. Still refer to FIG. 15a, FIG. 15b, FIG. 16a, and FIG. 16b. With reference to FIG. 17, impedance $R_{A\text{-}C} = R_1$, impedance $R_{B\text{-}C} = R_1 + R_0$. Therefore, the liver impedance $R_0 = R_{B\text{-}C} - R_{A\text{-}C}$. In an example, the liver impedance may also be referred to as a third physiological parameter.

It may be understood that, during measurement, when each group of electrodes on the wearable device 100 include one electrode, the user may touch the electrode with one finger; or when each group of electrodes on the wearable device 100 include two electrodes, the user may touch the electrodes with two fingers. In addition, after the two groups of electrodes on the wearable device 100 are conducted, for a principle of measuring impedance, refer to the foregoing descriptions about FIG. 10 and FIG. 11. Details are not described herein again.

It may be understood that, in FIG. 14, the left upper limb may also be replaced with the right upper limb. In this case, a measurement process is similar to that for the left upper limb, and details are not described herein again. In addition, an execution sequence of S1411 and S1412 may be randomly selected, which is not limited herein.

For example, as shown in FIG. 18, the method for measuring the liver impedance may further include the following steps:
S1801: Determine upper limb impedance.

Specifically, when determining the upper limb impedance, the user may simultaneously use the left hand and the right hand to contact different electrodes on the wearable device 100, so as to obtain the upper limb impedance.

For example, when two groups of electrodes are disposed on the wearable device 100, one group of electrodes are disposed on a rear part of the device body 11, and the other group of electrodes are disposed on the left side or the right side of the device body 11 (as shown in FIG. 1, FIG. 2, FIG. 3, or FIG. 4), in a process of measuring the upper limb impedance by using the wearable device 100, as shown in FIG. 19a, the user may touch the one group of electrodes on the left side of the device body 11 of the wearable device 100 with a finger of the left hand, and simultaneously touch the other group of electrodes on the rear part of the device body 11 of the wearable device 100 with a finger of the right hand. Further, after the user places the left finger and the right finger onto the electrodes of the wearable device 100, the group of electrodes in contact with the left hand of the user and the electrodes in contact with the right hand of the user are conducted. In this case, a current generated by the current generation unit 132 in the processor 13 of the wearable device 100 flows in a loop formed between the left hand and the right hand of the user. Then, the voltage measurement unit 132 in the processor 13 of the wearable device 100 may measure a voltage value between the electrode in contact with the left hand of the user and the electrode in contact with the right hand of the user. Finally, the control and processing unit 133 in the processor 13 of the wearable device 100 may obtain the upper limb impedance based on the voltage value measured by the voltage measurement unit 132 and a current value of the current generated by the current generation unit 131. In an example, the finger of the left hand of the user described in FIG. 19a may also be referred to as a first part, the finger of the right hand of the user described in FIG. 19a may also be referred to as a second part, and the upper limb impedance may also be referred to as a first physiological parameter.

S1802: Determine impedance from the left upper limb to the liver.

Specifically, when determining the impedance from the left upper limb to the liver, the user may contact some electrodes on the wearable device 100 with a finger of the left hand, and contact the other electrodes on the wearable device 100 with an area in a lower part of the liver on the user body, so as to obtain the impedance from the left upper limb to the liver.

For example, when two groups of electrodes are disposed on the wearable device 100, one group of electrodes are disposed on a rear part of the device body 11, and the other group of electrodes are disposed on the left side or the right side of the device body 11 (as shown in FIG. 1, FIG. 2, FIG. 3, or FIG. 4), in a process of measuring the impedance from the left upper limb to the liver by using the wearable device 100, as shown in FIG. 19b, the user may touch the group of electrodes on the left side of the device body 11 of the wearable device 100 with a finger of the left hand, and simultaneously place the other group of electrodes on the rear part of the device body 11 of the wearable device 100 onto the area in the lower part of the liver on the user body. Further, a group of electrodes that are in contact with the left hand of the user and an electrode that is in contact with the area in the lower part of the liver on the user body are conducted. In this case, a current generated by the current generation unit 132 in the processor 13 of the wearable device 100 flows in a loop formed between the left hand of the user and the area in the lower part of the liver on the user body. Then, the voltage measurement unit 132 in the processor 13 of the wearable device 100 may measure a voltage value between the electrode in contact with the left hand of the user and the electrode in contact with the area in the lower part of the liver on the user body. Finally, the control and processing unit 133 in the processor 13 of the wearable device 100 may obtain the impedance from the left upper limb to the liver based on the voltage value measured by the voltage measurement unit 132 and a current value of the current generated by the current generation unit 131. In an example, the area in the lower part of the liver described in FIG. 19b may also be referred to as a third part, and the impedance from the left upper limb to the liver may also be referred to as a second physiological parameter.

S1803: Determine impedance from the right upper limb to the liver.

Specifically, a process of determining the impedance from the right upper limb to the liver is similar to a process of determining the impedance from the left upper limb to the liver, except that the user contacts the electrode with a finger of the right hand in lieu of a finger of the left hand. For details, refer to the foregoing description. Details are not described herein again. In an example, the impedance from the right upper limb to the liver may also be referred to as a third physiological parameter.

S1804: Obtain the liver impedance based on the upper limb impedance, the impedance from the left upper limb to the liver, and the impedance from the right upper limb to the liver.

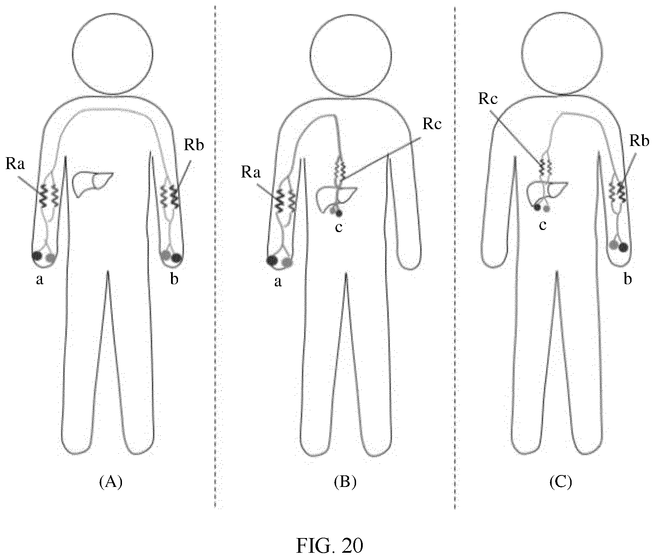

Specifically, after the upper limb impedance, the impedance from the left upper limb to the liver, and the impedance from the right upper limb to the liver are obtained, the liver impedance may be obtained based on the upper limb impedance, the impedance from the left upper limb to the liver, and the impedance from the right upper limb to the liver. For example, as shown in (A) in FIG. 20, the upper limb impedance is equivalent to impedance between a and b ($R_{a-b}$ for short), where the left upper limb impedance is equivalent to impedance $R_a$, the right upper limb impedance is equivalent to impedance $R_b$, and impedance $R_{a-b} = R_a + R_b$. As shown in (B) in FIG. 20, the impedance from the left upper limb to the liver is equivalent to impedance between a and c ($R_{a-c}$ for short), where the left upper limb impedance is equivalent to impedance $R_a$, the liver impedance is equivalent to impedance $R_c$, and impedance $R_{a-c} = R_a + R_c$. As shown in (C) in FIG. 20, the impedance from the right upper limb to the liver is equivalent to impedance between b and c ($R_{b-c}$ for short), where the right upper limb impedance is equivalent to impedance $R_b$, the liver impedance is equivalent to impedance $R_c$, and impedance $R_{b-c} = R_b + R_c$. Therefore, the liver impedance is equivalent to

$$R_c = \frac{(R_{a-c} + R_{b-c} - R_{a-b})}{2}$$

. In an example, the liver impedance may also be referred to as a fourth physiological parameter.

Further, after the liver impedance is measured, a liver fat content may be obtained based on the liver impedance, so as to evaluate a liver risk level.

For example, the liver fat content may be calculated by using the following formula. The formula (referred to as "Formula 15" below) is:

$$M = \omega_1 R + \frac{\omega_2}{R} + \omega_3 \quad \text{Formula 15}$$

M is the liver fat content, R is the liver impedance, and ($\omega_1$, $\omega_2$, and $\omega_3$ are preset coefficients, which may be obtained by experiment.

For example, the liver fat content may also be calculated with reference to a body parameter of the user such as the height. In this case, the liver fat content may also be calculated by using the following formula. The formula (referred to as "Formula 16" below) is:

$$M = \omega_1 R + \frac{\omega_2}{R} + \omega_3 H + \omega_4 \quad \text{Formula 16}$$

M is the liver fat content, R is the liver impedance, H is the height, and $\omega_1$, $\omega_2$, $\omega_3$, and $\omega_4$ are preset coefficients, which may be obtained by experiment.

For example, when the liver impedance is measured under a plurality of currents of different frequencies, the liver fat content may be calculated by using the following formula. The formula (referred to as "Formula 17" below) is:

$$M = \omega_1 R_1 + \frac{\omega_2}{R_1} + \omega_3 R_2 + \frac{\omega_4}{R_2} + \omega_5 \quad \text{Formula 17}$$

M is the liver fat content, $R_1$ and $R_2$ are the liver impedance measured under currents of different frequencies, and $\omega_1$, $\omega_2$, $\omega_3$, $\omega_4$, and $\omega_5$ are preset coefficients, which may be obtained by experiment. It may be understood that, when the number of liver impedance values measured under currents of different frequencies is greater than or equal

to 3, " $\omega_n R_n + \frac{\omega_{n+1}}{R_n}$ " may be added to Formula 17, where $R_n$ is liver impedance measured under a current of an $n^{th}$ frequency, and $\omega_n$ and $\omega_{n+1}$, which may be obtained by experiment, are preset coefficients corresponding to $R_n$.

For example, when the liver impedance is measured under a plurality of currents of different frequencies, and the liver fat content is calculated with reference to a body parameter of the user such as the height, the liver fat content may also be calculated by using the following formula. The formula (referred to as "Formula 18" below) is:

$$M = \omega_1 R_1 + \frac{\omega_2}{R_1} + \omega_3 R_2 + \frac{\omega_4}{R_2} + \omega_5 H + \omega_6 \quad \text{Formula 18}$$

M is the liver fat content, $R_1$ and $R_2$ are the liver impedance measured under currents of different frequencies, H is the height, and $\omega_1$, $\omega_2$, $\omega_3$, $\omega_4$, $\omega_5$, and $\omega_6$ are preset coefficients, which may be obtained by experiment. It may be understood that, when the number of liver impedance values measured under currents of different frequencies is greater than or equal to 3, " $\omega_n R_n + \frac{\omega_{n+1}}{R_n}$ " may be added to Formula 18, where $R_n$ is the liver impedance measured under a current of an $n^{th}$ frequency, and $\omega_n$ and $\omega_{n+1}$, which may be obtained by experiment, are preset coefficients corresponding to $R_n$.

After the liver fat content is obtained, the liver risk level of the user may be determined based on a correspondence between the liver fat content and the liver risk level. For example, a preset correspondence between the liver fat content and the liver risk level may be shown in Table 1. When the liver fat content is determined to be "5", it can be learned from Table 1 that the liver risk level is "Suspected".

**Table 1**

| Liver fat content | Liver risk level |
| --- | --- |
| 0-4 | Normal |
| 4-7 | Suspected |
| 7-10 | Medium to high |

The foregoing is a description of a related measurement principle when the wearable device 100 in the technical solution of this application is not worn on the user body. The following uses the case shown in FIG. 2 as an example to describe a process of measuring the liver risk level. The process of measuring the liver risk level may include: the process of measuring the liver impedance described in FIG. 14, and the process of measuring the liver impedance described in FIG. 18. The following describes the two processes separately.

(1) The process of measuring the liver impedance described in FIG. 14

Specifically, an application program (such as Huawei Health) related to liver fat measurement may be installed on the wearable device 100. As shown in (A) in FIG. 21, after the user opens the application program related to liver fat measurement on the wearable device 100 and starts the liver fat measurement, the wearable device 100 may display personal information of the user, such as the name and the height, where the personal information can be modified by the user. In (A) in FIG. 21, the user may tap an "OK" button in an area a1 to proceed to a next step. Then, as shown in (B) in FIG. 21, the wearable device 100 may display guide information b. The guide information b is mainly used to guide the user to attach an electrode on the wearable device 100 to a specific position on the user body, for example, attach a group of electrodes to a xiphoid process of the user body or a position below a right rib of the user body, and touch the other group of electrodes with a finger. The guide information b may be text information, voice information, graphic information, or a combination of any two or three of the text information, the voice information, and the graphic information. It may be understood that, when selecting a button on the wearable device 100, the user may select by tapping, by using voice, or by using a gesture. A specific choice is subject to an actual need. This is not limited herein. In addition, after the wearable device 100 displays the guide information b (that is, displays an interface shown in (B) in FIG. 21), the wearable device 100 may generate a current required for measurement. Moreover, the wearable device 100 may also generate a current required for measurement before displaying the guide information b. This is not limited herein.

During the measurement, the wearable device 100 may display an interface shown in (C) in FIG. 21. Then, after measuring human body impedance R of the user, the wearable device 100 may prompt, by using voice, through vibration, and/or the like, the user that the measurement is completed, and display an interface shown in (D) in FIG. 21, that is, display guide information c. The guide information c is mainly used to guide the user to attach an electrode on the wearable device 100 to a specific position on the user body, for example, attach a group of electrodes to a xiphoid process of the user body or a position below a right rib of the user body, and touch the other group of electrodes with a finger. An electrode placement position guided by the guide information c is different from an electrode placement position guided by the guide information b. For example, when the electrode placement position guided by the guide information b is the xiphoid process of the user body, the electrode placement position guided by the guide information c is the position below the right rib of the user body.

Then, the wearable device 100 may display an interface shown in (E) in FIG. 21. After the wearable device 100 measures human body impedance R of the user again, the wearable device 100 may determine the liver fat content based on the two impedance values. Then, after measuring the liver fat content of the user, the wearable device 100 may prompt, by using voice or by using both voice and text, the user that the measurement is completed, and display a measurement result, that is, display an interface shown in (F) in FIG. 21. In addition, after displaying the interface shown in (F) in FIG. 21, the wearable device 100 may also display related result interpretation, that is, display an interface shown in (G) in FIG. 21. For example, if the risk of fatty liver is high, the result can be interpreted as follows: You have a high risk of fatty liver. For the sake of your health, please strictly control your diet, especially your oil and alcohol intake, and it is recommended that you take aerobic exercise for at least one hour every day. If the risk of fatty liver is moderate, the result can be interpreted as follows: You have a moderate risk of fatty liver. For the sake of your health, please control your diet, especially your oil and alcohol intake, and it is recommended that you take aerobic exercise every day. If the risk of fatty liver is low, the result can be interpreted as follows: You have a low risk of fatty liver. For the sake of your health, please eat a balanced diet, exercise properly, and control your alcohol intake. In addition to displaying the measurement result, the wearable device 100 may also report the measurement result by using voice.

For example, when the liver impedance is measured, the liver impedance may be compared with a preset impedance range to determine whether the measured liver impedance is normal. If the liver impedance falls within the preset impedance range, the measured liver impedance is normal; otherwise, the measured liver impedance is abnormal. Further, if it is determined that the measured liver impedance is abnormal, the user may be prompted to measure again. For example, the user may be prompted by using voice to measure again. After prompting the user, the wearable device 100 may return to an interface shown in (B) in FIG. 21.

For example, on the interfaces shown in (C) and (E) in FIG. 21, the wearable device 100 may also verify the measured body impedance of the user to determine whether the measured body impedance is normal. When the body impedance is measured, the body impedance may be compared with a preset impedance range to determine whether the measured body impedance is normal. If the body impedance falls within the preset impedance range, the measured body impedance is normal; otherwise, the measured body impedance is abnormal. Further, if it is determined that the measured body impedance is abnormal, the user may be prompted to measure again. For example, the user may be prompted by using voice to measure again. For example, after prompting the user, the wearable device 100 may return to a previous interface of a current display interface. For example, if the current display interface is (C) in FIG. 21, the interface shown in (B) in FIG. 21 is returned; or if the current display interface is (E) in FIG. 21, the interface shown in (D) in FIG. 21 is returned. In addition, on the interface shown in (E) in FIG. 21, the wearable device 100 may further determine the liver fat content based on the two measured body impedance values, so as to obtain a liver risk level.

It may be understood that, during measurement, the wearable device 100 may display different interfaces at different measurement stages to prompt the user.

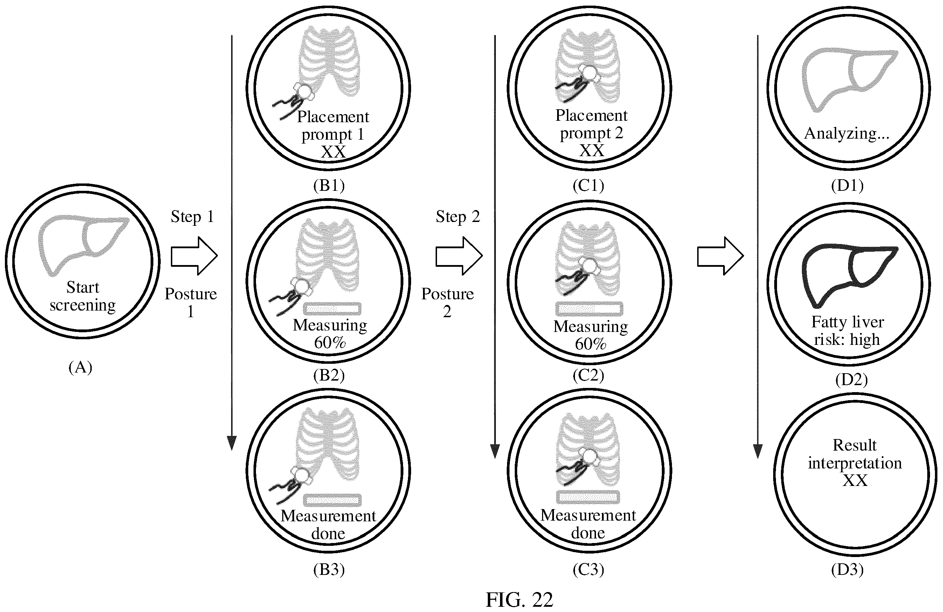

For example, after the user starts to measure the liver risk level, the wearable device 100 may display an interface shown in (A) in FIG. 22 to prompt the user to start fatty liver screening.

Then, the wearable device 100 may guide the user to use a posture 1 for measurement. In this process, the wearable device 100 may first display an interface shown in (B1) in FIG. 22 to visually prompt the user to place the wearable device 100 onto a preset position or area on the user body. A placement prompt may be the same as the guide information b in (B) in FIG. 21. After the user places the wearable device 100 onto the preset position or area on the user body, the wearable device 100 may display an interface shown in (B2) in FIG. 22 to prompt the user that body impedance is being measured in the posture 1. After the wearable device measures the body impedance of the user in the posture 1, the wearable device 100 may display an interface shown in (B3) in FIG. 22 to prompt the user that the body impedance measurement in the posture 1 has been completed. Then, the wearable device 100 may guide the user to use a posture 2 for measurement.

When the wearable device 100 guides the user to use the posture 2 for measurement, the wearable device 100 may first display an interface shown in (C1) in FIG. 22 to visually prompt the user to place the wearable device 100 onto a preset position or area on the user body. A placement prompt may be the same as the guide information c in (D) in FIG. 21. After the user places the wearable device 100 onto the preset position or area on the user body, the wearable device 100 may display an interface shown in (C2) in FIG. 22 to prompt the user that body impedance is being measured in the posture 2. After the wearable device measures the body impedance of the user in the posture 2, the wearable device 100 may display an interface shown in (C3) in FIG. 22 to prompt the user that the body impedance measurement in the posture 2 has been completed.

After measuring the body impedance of the user in different postures, the wearable device 100 may display an interface shown in (D1) in FIG. 22 to prompt the user that the liver risk level is being measured. After measuring the liver risk level, the wearable device 100 may display an interface shown in (D2) in FIG. 22 to display the liver risk level. Then, the wearable device 100 may display an interface shown in (D3) in FIG. 22 to display result interpretation of this measurement. The result interpretation may be the same as the result interpretation in (G) in FIG. 20. It should be noted that display interfaces shown in FIG. 22 are merely examples for description, and do not constitute a limitation on this solution. The display interfaces may be selected or re-drawn as required. This is not limited herein.

(2) The process of measuring the liver impedance described in FIG. 18

Specifically, an application program (such as Huawei Health) related to liver fat measurement may be installed on the wearable device 100. As shown in (A) in FIG. 23, after the user opens the application program related to liver fat measurement on the wearable device 100 and starts the liver fat measurement, the wearable device 100 may display personal information of the user, such as the name and the height, where the personal information can be modified by the user. In (A) in FIG. 23, the user may tap an "OK" button in an area a1 to proceed to a next step. Then, as shown in (B) in FIG. 23, the wearable device 100 may display prompt information a for measuring upper limb impedance, that is, enter a process of measuring the upper limb impedance. The prompt information a may be text information, voice information, graphic information, or a combination of any two or three of the text information, the voice information, and the graphic information. It may be understood that, when selecting a button on the wearable device 100, the user may select by tapping, by using voice, or by using a gesture. A specific choice is subject to an actual need. This is not limited herein. In addition, after the wearable device 100 displays the prompt information a (that is, displays an interface shown in (B) in FIG. 23), the wearable device 100 may generate a current required for measurement. Moreover, the wearable device 100 may also generate a current required for measurement before displaying the prompt information a. This is not limited herein.

In a process of measuring the upper limb impedance, the wearable device 100 may display an interface shown in (C) in FIG. 23. Then, after measuring the upper limb impedance of the user, the wearable device 100 may prompt, by using voice, through vibration, and/or the like, the user that the measurement is completed, and display an interface shown in (D) in FIG. 23, that is, display prompt information b for measuring liver-left upper limb impedance, that is, enter a process of measuring the liver-left upper limb impedance.

In a process of measuring the liver-left upper limb impedance, the wearable device 100 may display an interface shown in (E) in FIG. 23. After measuring the liver-left upper limb impedance of the user, the wearable device 100 may prompt, by using voice, through vibration, and/or the like, the user that the measurement is completed, and display an interface shown in (F) in FIG. 23, that is, display prompt information c for measuring liver-right upper limb impedance, that is, enter a process of measuring the liver-right upper limb impedance.

In a process of measuring the liver-right upper limb impedance, the wearable device 100 may display an interface shown in (G) in FIG. 23. After the wearable device 100 measures the liver-right upper limb impedance of the user, the wearable device 100 may determine the liver fat content based on the upper limb impedance, the liver-left upper limb impedance, and the liver-right upper limb impedance.

After measuring the liver fat content of the user, the wearable device 100 may prompt, by using voice or by using both voice and text, the user that the measurement is completed, and display a measurement result, that is, display an interface shown in (H) in FIG. 23. In addition, after displaying the interface shown in (H) in FIG. 23, the wearable device 100 may also display related result interpretation, that is, display an interface shown in (I) in FIG. 23. For example, if the risk of fatty liver is high, the result can be interpreted as follows: You have a high risk of fatty liver. For the sake of your health, please strictly control your diet, especially your oil and alcohol intake, and it is recommended that you take aerobic exercise for at least one hour every day. If the risk of fatty liver is moderate, the result can be interpreted as follows: You have a moderate risk of fatty liver. For the sake of your health, please control your diet, especially your oil and alcohol intake, and it is recommended that you take aerobic exercise every day. If the risk of fatty liver is low, the result can be interpreted as follows: You have a low risk of fatty liver. For the sake of your health, please eat a balanced diet, exercise properly, and control your alcohol intake. In addition to displaying the measurement result, the wearable device 100 may also report the measurement result by using voice.

It should be noted that display interfaces shown in FIG. 23 are merely examples for description, and do not constitute a limitation on this solution. The display interfaces may be selected or re-drawn as required, or new display interfaces may be added as required. This is not limited herein. In addition, an execution sequence of steps in FIG. 23 may be selected in an adaptive manner. This is not limited herein.

It may be understood that, in this embodiment of this application, in addition to displaying the liver risk level, the wearable device 100 may further display a physiological parameter of the user such as a body fat rate, so that the user can learn of the physiological parameter such as the body fat rate.

It may be understood that, a processor in embodiments of this application may be a central processing unit (central processing unit, CPU), another general-purpose processor, a digital signal processor (digital signal processor, DSP), an application-specific integrated circuit (application-specific integrated circuit, ASIC), a field programmable gate array (field programmable gate array, FPGA) or another programmable logic device, a transistor logic device, a hardware component, or any combination thereof. The general-purpose processor may be a microprocessor or any regular processor.

The method steps in embodiments of this application may be implemented by using hardware, or may be implemented through execution of software instructions by a processor. The software instructions may include corresponding software modules. The software modules may be stored in a random access memory (random access memory, RAM), a flash memory, a read-only memory (read-only memory, ROM), a programmable read-only memory (programmable ROM, PROM), an erasable programmable read-only memory (erasable PROM, EPROM), an electrically erasable programmable read-only memory (electrically EPROM, EEPROM), a register, a hard disk, a removable hard disk, a CD-ROM, or any other form of storage medium well-known in the art. For example, a storage medium is coupled to a processor, so that the processor can read information from the storage medium and write information into the storage medium. Certainly, the storage medium may be a component of the processor. The processor and the storage medium may be disposed in an ASIC.

The foregoing embodiments may be wholly or partially implemented by using software, hardware, firmware, or any combination thereof. When software is used to implement the embodiments, the embodiments may be wholly or partially implemented in a form of a computer program product. The computer program product includes one or more computer instructions. When the computer program instructions are loaded and executed on a computer, processes or functions according to embodiments of this application are wholly or partially generated. The computer may be a general-purpose computer, a dedicated computer, a computer network, or any other programmable apparatus. The computer instructions may be stored in a computer-readable storage medium, or may be transmitted by using the computer-readable storage medium. The computer instructions may be transmitted from a website, computer, server, or data center to another website, computer, server, or data center in a wired (for example, a coaxial cable, an optical fiber, or a digital subscriber line (DSL)) or wireless (for example, infrared, radio, or microwave) manner. The computer-readable storage medium may be any usable medium accessible by the computer, or a data storage device, such as a server or a data center, integrating one or more usable media. The usable medium may be a magnetic medium (for example, a floppy disk, a hard disk, or a magnetic tape), an optical medium (for example, a DVD), a semiconductor medium (for example, a solid-state disk (solid-state disk, SSD)), or the like.

It may be understood that numbers in embodiments of this application are merely used for differentiation for ease of description, and are not used to limit the scope of embodiments of this application.

## Claims

1. A physiological parameter measurement method, wherein the method is applied to a wearable device, and the wearable device comprises a device body, wherein a display screen, a first group of electrodes, and a second group of electrodes are disposed on the device body, the first group of electrodes are located on a side that is on the device body but is opposite to the display screen, and the second group of electrodes are located on a lateral side of the device body; and the method comprises:

   detecting a first operation;
   displaying a first user interface in response to the first operation, wherein the first user interface indicates a user to respectively contact a first part and a second part with the first group of electrodes and the second group of electrodes, to conduct the first group of electrodes and the second group of electrodes;
   generating a first current when the first group of electrodes and the second group of electrodes are conducted, wherein the first current has a first current value;
   determining a first voltage value between the first group of electrodes and the second group of electrodes;
   determining a first physiological parameter based on the first current value and the first voltage value;
   displaying a second user interface, wherein the second user interface indicates the user to respectively contact the first part and a third part with the first group of electrodes and the second group of electrodes, to conduct the first group of electrodes and the second group of electrodes;
   generating a second current when the first group of electrodes and the second group of electrodes are conducted, wherein the second current has a second current value;
   determining a second voltage value between the first group of electrodes and the second group of electrodes;
   determining a second physiological parameter based on the second current value and the second voltage value;

and

determining a third physiological parameter based on the first physiological parameter and the second physiological parameter.

2. The method according to claim 1, wherein after the determining a second physiological parameter based on the second current value and the second voltage value, the method further comprises: displaying a third user interface, wherein the third user interface indicates the user to respectively contact the second part and the third part with the first group of electrodes and the second group of electrodes, to conduct the first group of electrodes and the second group of electrodes;

generating a third current when the first group of electrodes and the second group of electrodes are conducted, wherein the third current has a third current value;
determining a third voltage value between the first group of electrodes and the second group of electrodes; and determining a fourth physiological parameter based on the third current value and the third voltage value; and the determining a third physiological parameter based on the first physiological parameter and the second physiological parameter specifically comprises: determining the third physiological parameter based on the first physiological parameter, the second physiological parameter, and the fourth physiological parameter.

3. The method according to claim 1 or 2, wherein the method comprises: generating a target current, wherein the target current has a target current value;

determining a target voltage value between the first group of electrodes and the second group of electrodes; and determining a target physiological parameter based on the target current value and the target voltage value; and the method further comprises:

generating currents of m frequencies, wherein m is a positive integer greater than or equal to 2, currents of different frequencies are generated at different moments, and current values of the currents of different frequencies each are the target current value;
determining voltage values between the first group of electrodes and the second group of electrodes under the currents of different frequencies, to obtain m voltage values, wherein one voltage value corresponds to a current of one frequency; and
determining the target physiological parameter based on the current value of the target current and the m voltage values, wherein
the target current is the first current, the target current value is the first current value, the target voltage value is the first voltage value, and the target physiological parameter is the first physiological parameter; or the target current is the second current, the target current value is the second current value, the target voltage value is the second voltage value, and the target physiological parameter is the second physiological parameter; or the target current is the third current, the target current value is the third current value, the target voltage value is the third voltage value, and the target physiological parameter is the fourth physiological parameter.

4. The method according to any one of claims 1 to 3, wherein the second group of electrodes are integrated in a physical button on the lateral side of the device body.

5. The method according to any one of claims 1 to 4, wherein the first group of electrodes comprise one electrode, and the second group of electrodes comprise one electrode; or the first group of electrodes comprise two electrodes, and the second group of electrodes comprise two electrodes.

6. The method according to claim 5, wherein the second group of electrodes comprise a first electrode and a second electrode, and the first electrode and the second electrode are located on a same side of the device body.

7. A wearable device, comprising:

a device body, wherein a display screen, a first group of electrodes, and a second group of electrodes are disposed on the device body, the first group of electrodes are located on a side that is on the device body but is opposite to the display screen, and the second group of electrodes are located on a lateral side of the device body;
a memory, wherein the memory stores a computer program; and

a processor, wherein the processor is electrically connected to the first group of electrodes and the second group of electrodes, wherein

when the computer program stored in the memory is executed by the processor, the wearable device is enabled to perform the method according to any one of claims 1 to 6.

8. The wearable device according to claim 7, wherein the second group of electrodes are integrated in a physical button on the lateral side of the device body.

9. The wearable device according to claim 7 or 8, wherein the first group of electrodes comprise one electrode, and the second group of electrodes comprise one electrode; or the first group of electrodes comprise two electrodes, and the second group of electrodes comprise two electrodes.

10. The wearable device according to claim 9, wherein the second group of electrodes comprise a first electrode and a second electrode, and the first electrode and the second electrode are located on a same side of the device body.

11. A computer storage medium, wherein the computer storage medium stores instructions, and when the instructions are run on a computer, the computer is enabled to perform the method according to any one of claims 1 to 6.

12. A computer program product comprising instructions, wherein when the instructions are run on a computer, the computer is enabled to perform the method according to any one of claims 1 to 6.

FIG. 1

FIG. 2

FIG. 3

FIG. 4

FIG. 5

FIG. 6

FIG. 7

FIG. 8

FIG. 9

(A)

(B)

FIG. 10

(A)

(B)

FIG. 11

(A)

Name: U001
Height: 175 cm
Weight: 78 kg
Waist circumference:
90 cm
OK
a1

(B)

Guide information
XX

(C)

Measuring
60%

(D)

Fatty liver risk: high

(E)

Result
interpretation
XX

FIG. 12

S1301

Obtain a first physiological parameter of a user through measurement by a wearable device

S1302

Determine n second physiological parameters based on the first physiological parameter, where n is a positive integer greater than or equal to 1

S1303

Determine whether the user enters a waist circumference

S1304

No → Calculate a liver fat content by using a model 1

Yes

S1305

Calculate a liver fat content by using a model 2

FIG. 13a

S1301

Obtain a first physiological parameter of a user through measurement by a wearable device

S1302

Determine n second physiological parameters based on the first physiological parameter, where n is a positive integer greater than or equal to 1

S1304

Calculate a liver fat content by using a model 1

FIG. 13b

S1300

Prompt a user to enter a waist circumference

S1301

Obtain a first physiological parameter of the user through measurement by a wearable device

S1302

Determine n second physiological parameters based on the first physiological parameter, where n is a positive integer greater than or equal to 1

S1305

Calculate a liver fat content by using a model 2

FIG. 13c

S1401

Determine first impedance from the left upper limb to an upper part of the liver

S1402

Determine second impedance from the left upper limb to a lower part of the liver

S1403

Obtain liver impedance based on the first impedance and the second impedance

FIG. 14

FIG. 15a

FIG. 15b

FIG. 16a

FIG. 16b

FIG. 17

| Determine upper limb impedance | S1801 |
| Determine impedance from the left upper limb to the liver | S1802 |
| Determine impedance from the right upper limb to the liver | S1803 |
| Obtain liver impedance based the upper limb impedance, the impedance from the left upper limb to the liver, and the impedance from the right upper limb to the liver | S1804 |

FIG. 18

FIG. 19a

FIG. 19b

FIG. 20

Name: a
Height: 170 cm

a1

OK

(A)

Guide
information b:
XX

(B)

Measuring
50%

(C)

Measurement
result:
XX

(F)

Measuring
49%

(E)

Guide
information c:
XX

(D)

Result
interpretation:
XX

(G)

FIG. 21

FIG. 22

EP 4 344 623 A1

(A)

Name: a
Height: 170 cm

a1 — OK

(B)

Upper limb
impedance
Prompt
information a

(C)

Measuring
50%

(F)

Liver–right
upper limb
impedance
Prompt
information c

(E)

Measuring
49%

(D)

Liver–left
upper limb
impedance
Prompt
information b

(G)

Measuring
49%

(H)

Measurement
result:
XX

(I)

Result
interpretation:
XX

FIG. 23

## INTERNATIONAL SEARCH REPORT

| | International application No. |
|---|---|
| | **PCT/CN2022/102467** |

**A.    CLASSIFICATION OF SUBJECT MATTER**

A61B 5/0537(2021.01)i;  A61B 5/053(2021.01)i

According to International Patent Classification (IPC) or to both national classification and IPC

**B.    FIELDS SEARCHED**

Minimum documentation searched (classification system followed by classification symbols)

A61B

Documentation searched other than minimum documentation to the extent that such documents are included in the fields searched

Electronic data base consulted during the international search (name of data base and, where practicable, search terms used)

CNTXT, ENTXT, ENTXTC, DWPI, CNKI: 阻抗, 电阻, 电流, 电压, 电极, 组, 对, 肝, 内脏, 脂肪, 臂, 手, 上肢, 腕, 剑突, 肋骨, 腰, 胸, 腹, impedance, resistance, electric, current, voltage, electrode?, pair?, group?, coupl+, liver, viscera?, fat, arm?, hand?, finger?, upper, limb?, wrist?, xiphoid+, rib+, costa, waist?, chest, thorax, breast, abdomen, ventral, belly

**C.    DOCUMENTS CONSIDERED TO BE RELEVANT**

| Category* | Citation of document, with indication, where appropriate, of the relevant passages | Relevant to claim No. |
|---|---|---|
| PX | CN 114431826 A (HUAWEI TECHNOLOGIES CO., LTD.) 06 May 2022 (2022-05-06) description, paragraphs 254-281, and figures 18 and 25-29 | 1-12 |
| X | WO 2018201629 A1 (HUAWEI TECHNOLOGIES CO., LTD.) 08 November 2018 (2018-11-08) description, page 9, line 4 - page 21, line 37, and figures 1-33 | 1-12 |
| X | US 6567692 B1 (YAMATO SCALE CO., LTD.) 20 May 2003 (2003-05-20) description, column 19, line 17 to column 21, line 53, and figure 7 | 1-12 |
| X | JP 2001000411 A (MATSUSHITA ELECTRIC INDUSTRIAL CO., LTD.) 09 January 2001 (2001-01-09) description, paragraphs 12-31, and figures 1-3 | 1-12 |
| X | CN 1903120 A (ZHEJIANG UNIVERSITY OF TECHNOLOGY) 31 January 2007 (2007-01-31) description, page 10 - page 16, and figures 1-5 | 1-12 |
| X | US 5579782 A (OMRON TATEISI ELECTRONICS CO.) 03 December 1996 (1996-12-03) description, column 14, line 65 - column 16, line 64, and figures 1-2 and 19-23 | 1-12 |

☑ Further documents are listed in the continuation of Box C.     ☑ See patent family annex.

| | |
|---|---|
| *    Special categories of cited documents: | "T"    later document published after the international filing date or priority date and not in conflict with the application but cited to understand the principle or theory underlying the invention |
| "A"    document defining the general state of the art which is not considered to be of particular relevance | |
| "E"    earlier application or patent but published on or after the international filing date | "X"    document of particular relevance; the claimed invention cannot be considered novel or cannot be considered to involve an inventive step when the document is taken alone |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
|---|---|
| **17 August 2022** | **07 September 2022** |

| Name and mailing address of the ISA/CN | Authorized officer |
|---|---|
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

International application No.

**PCT/CN2022/102467**

### C. DOCUMENTS CONSIDERED TO BE RELEVANT

| Category* | Citation of document, with indication, where appropriate, of the relevant passages | Relevant to claim No. |
|---|---|---|
| A | CN 1608586 A (OMRON HEALTHCARE CO., LTD.) 27 April 2005 (2005-04-27) entire document | 1-12 |
| A | JP 2007159699 A (TANITA SEISAKUSHO K. K.) 28 June 2007 (2007-06-28) entire document | 1-12 |

Form PCT/ISA/210 (second sheet) (January 2015)

**INTERNATIONAL SEARCH REPORT**
Information on patent family members

International application No.

**PCT/CN2022/102467**

| Patent document cited in search report | | | Publication date (day/month/year) | Patent family member(s) | | | Publication date (day/month/year) |
|---|---|---|---|---|---|---|---|
| CN | 114431826 | A | 06 May 2022 | WO | 2022088921 | A1 | 05 May 2022 |
| WO | 2018201629 | A1 | 08 November 2018 | CN | 110573073 | A | 13 December 2019 |
| US | 6567692 | B1 | 20 May 2003 | WO | 0124694 | A1 | 12 April 2001 |
| | | | | JP | 2001104271 | A | 17 April 2001 |
| | | | | EP | 1138258 | A1 | 04 October 2001 |
| | | | | DE | 60019551 | D1 | 25 May 2005 |
| | | | | CN | 1327375 | A | 19 December 2001 |
| | | | | EP | 1138258 | A4 | 11 June 2003 |
| | | | | CN | 1182814 | C | 05 January 2005 |
| | | | | EP | 1138258 | B1 | 20 April 2005 |
| | | | | DE | 60019551 | T2 | 23 February 2006 |
| | | | | JP | 4357049 | B2 | 04 November 2009 |
| JP | 2001000411 | A | 09 January 2001 | None | | | |
| CN | 1903120 | A | 31 January 2007 | CN | 100393276 | C | 11 June 2008 |
| US | 5579782 | A | 03 December 1996 | US | 6308096 | B1 | 23 October 2001 |
| | | | | US | 6088615 | A | 11 July 2000 |
| | | | | US | 6321112 | B1 | 20 November 2001 |
| | | | | JP | H11285479 | A | 19 October 1999 |
| | | | | JP | H11285480 | A | 19 October 1999 |
| | | | | JP | 3171248 | B2 | 28 May 2001 |
| | | | | JP | 3175723 | B2 | 11 June 2001 |
| | | | | JP | 2001157672 | A | 12 June 2001 |
| | | | | JP | 3182991 | B2 | 03 July 2001 |
| | | | | JP | 3211117 | B2 | 25 September 2001 |
| | | | | JP | 3211118 | B2 | 25 September 2001 |
| | | | | JP | 3414378 | B2 | 09 June 2003 |
| CN | 1608586 | A | 27 April 2005 | US | 2005090760 | A1 | 28 April 2005 |
| | | | | JP | 2005124914 | A | 19 May 2005 |
| | | | | CN | 100443047 | C | 17 December 2008 |
| JP | 2007159699 | A | 28 June 2007 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110770421